# EUROPEAN PATENT APPLICATION

(11) **EP 3 031 427 A1**
(43) Date of publication of application: **15.06.2016**
(21) Application number: 14833942.7
(22) Date of filing: 06.08.2014
(51) Int. Cl.: A61F 5/02, A61F 5/56

(54) **AIRWAY EXPANSION DEVICE**

(30) Priority: 06.08.2013 JP 2013163284
(71) Applicant: Fuso Pharmaceutical Industries, Ltd., Osaka-shi, Osaka 541-0045 (JP); Nextier, Toyohashi-shi, Aichi 441-8107 (JP)
(72) Inventor: SHINZATO, Toru, Nagoya-shi Aichi 462-0841 (JP); KITAHARA, Yuzuru, Osaka-shi, Osaka 536-8523 (JP)
(74) Representative: Schwabe - Sandmair - Marx
(86) International application number: PCT/JP2014/070668
(87) International publication number: WO 2015/020070

(57) **Abstract**

An airway is secured without obstructing sleep even when the lingual radix falls into the airway. An airway expansion apparatus (1) is configured to include a pillow portion (2) that supports a cranial portion, a pair of left and right mandible holding portions (3) that hold the mandible coupled to the cranial portion at the temporomandibular joint, a first actuation mechanism (4) that causes the mandible holding portions to come into contact with the mandible, and a second actuation mechanism (5) that lifts the mandible holding portions with respect to the pillow portion with the mandible holding portions kept in contact with the mandible, in which the mandible is kept lifted.

## Description

### Technical Field

The present invention relates to an airway expansion apparatus that secures the airway.

### Background Art

In recent years, sleep apnea syndromes are being currently at issue as disease in which respiratory arrest (apnea) lasting for ten or more seconds is repeated a plurality of times during sleep. This sleep apnea syndrome is caused by sagging of the lingual radix during third-stage and fourth stage sleep which are great in depth during non-REM sleep, thereby obstructing the airway. In an apnea condition, a thoracic cavity internal pressure acts as a strong negative pressure, causing blood to accumulate in the thoracic cavity which causes a high blood pressure at wake-up time, cardiac disease or the like. Furthermore, since deep sleep is obstructed by the sleep apnea syndrome, patients feel sleepy during the daytime and get distracted, which may cause an accident or the like.

Apnea during sleep occurs when a person is sleeping lying on his/her back, the lingual radix falls into the airway under its own weight, obstructing the airway in the vicinity of the throat. The airway in the vicinity of the throat is surrounded by a tissue formed of muscle, fat or the like, the frontal tissue of the airway including the tongue is adhered to the submaxilla and the rear tissue is adhered to the cranial bone via the cervical bone. For this reason, when the person is sleeping in a supine position with the face facing upward, if the submaxilla is caused to move upward or in the upward and lower limb direction, that is, diagonally upward direction, the frontal wall of the airway moves upward, the airway expands, and therefore even when the lingual radix falls into the airway, the airway is still secured.

Conventionally, airway expansion apparatuses are known which secure the airway of a person sleeping in a supine position with the face facing upward, by allowing the person to push the submaxilla out in the lower limb and upward direction with respect to the cranial bone, and thereby prevent apnea during sleep (e.g., see Patent Literature 1). When the head portion having the cranial bone as a skeletal frame is defined as a cranial portion, the airway expansion apparatus described in Patent Literature 1 is attached to the cranial portion just like a headgear, causes a pair of left and right fixed holding portions to hold the temporal bone of the cranial bone and at the same time causes a pair of left and right movable holding portions to hold the submaxilla coupled to the cranial bone at the temporomandibular joint. With this airway expansion apparatus, each movable holding portion moves apart from each fixed holding portion, causing the submaxilla to be pushed out in the lower limb and upward direction with respect to the cranial bone, causing the front wall of the airway to move upward which is the direction in which the face is oriented while sleeping in the supine position, expanding the airway, thus securing the airway even when the lingual radix falls into the airway.

### Citation List

### Patent Literature

[Patent Literature 1] Japanese Patent Application Laid-Open No.2011-072733

### Summary of Invention

### Technical Problem

However, since the airway expansion apparatus described in Patent Literature 1 places a fulcrum on the cranial portion when pushing out the submaxilla in the lower limb and upward direction, it is necessary to cause one end of the airway expansion apparatus to come into close contact with the cranial portion in advance and reliably fix it before the person going to sleep. For this reason, the airway expansion apparatus described in Patent Literature 1 requires a burdensome operation of attaching the apparatus. Furthermore, when the airway expansion apparatus is attached, the cranial portion is fastened with a belt, and therefore there is a problem that the user always has to start to sleep while feeling oppression and cannot obtain comfortable sleep.

The present invention has been implemented in view of the above-described points and it is an object of the present invention to provide an airway expansion apparatus capable of securing the airway without disturbing sleep even when the lingual radix falls into the airway.

### Solution to Problem

An airway expansion apparatus according to the present invention is provided with a pillow portion that supports a cranial portion, a pair of left and right mandible holding portions that hold a mandible coupled to the cranial portion at a temporomandibular joint, a first actuation mechanism that causes the mandible holding portions to come into contact with the mandible, and a second actuation mechanism that lifts the mandible holding portions with respect to the pillow portion with the mandible holding portions kept in contact with the mandible.

According to this configuration, the first actuation mechanism first causes the pair of left and right mandible holding portions to hold the mandible and then the second actuation mechanism causes the mandible to remain lifted. It is therefore possible to prevent a sleep apnea syndrome and avoid a burdensome operation of bringing one end of the airway expansion apparatus into close contact with the cranial portion in advance before the person going to sleep and reliably fixing the apparatus. Moreover, only the submaxilla is held and the cranial portion is not fastened, which prevents the user from feeling oppression and allows the user to enjoy a comfortable sleep.

The airway expansion apparatus of the present invention is provided with pads in close contact with the mandible, and the mandible holding portions hold the mandible via the pads. According to this configuration, the mandible is held by the mandible holding portions via the pads, and it is thereby possible to prevent dislocation of the mandible with respect to the mandible holding portions.

In the airway expansion apparatus of the present invention, a contact surface of the pad in close contact with the mandible is curved so as to follow a corner of the mandible. According to this configuration, the curved portion of the pad is caught on the corner of the mandible, thus strengthening unity between the mandible and the mandible holding portions.

In the airway expansion apparatus of the present invention, the contact surface of the pad in close contact with the mandible has adhesiveness. This configuration can prevent dislocation of the mandible with respect to the contact surface of the pad.

In the airway expansion apparatus of the present invention, the mandible holding portions are fixed to the first actuation mechanism, the first actuation mechanism is detachably connected to an actuator of the second actuation mechanism via the mandible holding portions, and the first actuation mechanism can be removed from the second actuation mechanism by removing the mandible holding portions from the second actuation mechanism. According to this configuration, the mandible holding portions and the first actuation mechanism are attached to the mandible before the user going to sleep, the first actuation mechanism and the second actuation mechanism are connected together via the mandible holding portions, and the second actuation mechanism acts so as to lift the mandible holding portions. As described above, the airway expansion apparatus can be made simpler.

In the airway expansion apparatus of the present invention, the second actuation mechanism uses compressed air as a drive source. According to this configuration, it is possible to automatically lift the mandible and easily secure the airway.

The airway expansion apparatus of the present invention includes a weight portion coupled to the mandible holding portions and a conversion mechanism that converts gravity of the weight portion into a direction in which the mandible holding portions are lifted, and the gravity of the weight portion is used as a drive source. According to this configuration, it is possible to lift the mandible holding portions using the gravity of the weight portion. For this reason, it is possible to keep the mandible lifted in a low-cost configuration without using any drive source such as an air pump.

In the airway expansion apparatus of the present invention, the pillow portion includes a cushioning portion that supports the cranial portion and a body portion that accommodates the cushioning portion and is formed into a U-figured shape so as to oscillate in a crosswise direction, and the first actuation mechanism and the second actuation mechanism are attached to the body portion. According to this configuration, since the body portion can oscillate, it is possible to easily change the body position from a supine position to a lateral position or from a lateral position to a supine position, never preventing roll over during sleep.

In the airway expansion apparatus of the present invention, the second actuation mechanism is provided with a switch mechanism that causes the pillow portion to support the cranial portion when the angle of oscillation of the body portion is within a predetermined angle range, switches, when the user is sleeping in a supine position, to an operating state in which the mandible holding portions are lifted in a lower limb and upward direction, where the direction in which the face is facing is assumed to be an upward direction, and switches, when the angle of oscillation of the body portion is outside the predetermined angle range, to a released state in which the operating state is released. According to this configuration, the predetermined angle range is adjusted according to the postural change between a supine position and a lateral position, and it is thereby possible to lift the mandible only in a supine position or a posture close to a supine position in which sleep apnea occurs.

In the airway expansion apparatus of the present invention, the switch mechanism includes a pair of left and right switches provided in correspondence with the predetermined angle range and a pendulum portion that oscillates between the pair of left and right switches, and the released state is selected when the pendulum portion comes into contact with any one of the pair of left and right switches and the operating state is selected when the pendulum portion detaches from the pair of left and right switches. According to this configuration, it is possible to mechanically change the switch according to the posture during sleep.

An airway expansion apparatus according to the present invention is provided with a pillow portion that supports a cranial portion, a pair of left and right mandible holding portions that hold a mandible coupled to the cranial portion at a temporomandibular joint, and a pair of left and right side plates in which slopes are formed so as to elevate from the mandible toward the parietal region in correspondence with the mandible holding portions, in which the mandible holding portion includes an abutting portion abutting on the slope and the abutting portion moves the mandible holding portion in a direction perpendicular to the slope by abutting on the slope. According to this configuration, when the user places his/her cranial portion on the pillow portion, the mandible holding portions are pushed out in a direction perpendicular to the slope as the abutting portion moves downward along the slopes of the pair of left and right side plates. Thus, the mandible holding portions can be lifted using the weight of the cranial portion and there is no need to use any drive source such as an air pump, thereby providing a low-cost airway expansion apparatus.

In the airway expansion apparatus of the present invention, the pad is a bag body filled with a mixture of grains and an adhesive substance. According to this configuration, when the pad is pushed against the mandible before using the pad, the pad is deformed into the shape of the mandible, and if the pad is then detached from the mandible and left as is, the pad remains deformed into the shape of the mandible and is hardened as the adhesive substance coagulates.

An airway expansion apparatus of the present invention is provided with a mandible holding unit including a pair of mandible holding portions that hold a mandible coupled to a cranial portion at a temporomandibular joint and a coupling portion that couples the pair of mandible holding portions and keeps the mandible holding portions in contact with the mandible, and an actuator that supports the cranial portion, is attached to the mandible holding portions and lifts the mandible holding portions with respect to the cranium supporting portion with the mandible holding portions kept in contact with the mandible, in which the actuator is made up of a bag body whose interior can be filled with a fluid and the fluid filling the interior of the bag body can flow. According to this configuration, when the cranial portion is placed on the bag body, the bag body is crushed under the own weight of the cranial portion and the fluid in the bag body is caused to flow, thereby causing the bag body to deform. The deformation of the bag body causes the mandible holding unit coupled to the bag body to move. The movement of the mandible holding unit causes the mandible held by the mandible holding unit to be lifted relative to the cranial portion, allowing the airway to be expanded. Using the flow of the fluid in this way can implement an airway expansion apparatus in a low-cost configuration without requiring any complicated configuration.

In the airway expansion apparatus of the present invention, the actuator is attachable/detachable to/from the mandible holding unit. According to this configuration, the mandible holding portions and the actuator can be attached separately, thereby making it possible to simplify a burdensome operation of attaching the airway expansion apparatus.

In the airway expansion apparatus of the present invention, the actuator is detachable. According to this configuration, it is possible to attach the mandible holding portions and the actuator to the mandible in advance before the user going to sleep and couple the actuator before placing the cranial portion on the cranium supporting portion. For this reason, it is possible to simplify a burdensome operation of attaching the airway expansion apparatus.

In the airway expansion apparatus of the present invention, the volume of a region of the actuator attached to the mandible holding portion is smaller than the volume of a region of the actuator that supports the cranial portion. According to this configuration, when the cranial portion is placed on the cranium supporting portion, a sufficient amount of fluid moves from the region of the actuator that supports the cranial portion to the region attached to the mandible holding portions, and therefore the protruding sections expand and can lift the mandible holding portions upward. Thus, it is possible to achieve effective airway expansion

### Advantageous Effects of Invention

According to the present invention, it is possible to keep the mandible lifted in a diagonally upward direction in a posture with the cranial portion being supported by the pillow portion and secure the airway even if the lingual radix falls into the airway without obstructing sleep.

### Brief Description of Drawings

FIG. 1 is a top view of an airway expansion apparatus according to the present embodiment;
FIG. 2 is a side view of the airway expansion apparatus according to the present embodiment;
FIG. 3 is a side view of the airway expansion apparatus according to the present embodiment seen from the parietal region side;
FIG. 4 is a cross-sectional perspective view of a pad according to the present embodiment;
FIG. 5 is a mid-sagittal view of the tongue, oral cavity, nasal cavity and airway during sleep in a supine position;
FIG. 6 is a side view of the cranial portion made up of the submaxilla, cranial bone and temporomandibular joint in a supine position with the cranial portion being supported by the pillow portion;
FIG. 7 is an operation diagram illustrating a mandible holding operation of the airway expansion apparatus according to the present embodiment;
FIG. 8 is an operation diagram illustrating a mandible lifting operation of the airway expansion apparatus according to the present embodiment;
FIG. 9 is an operation diagram illustrating a switching operation of a switch mechanism of the airway expansion apparatus according to the present embodiment;
FIG. 10 is an operation diagram illustrating an airway expansion apparatus according to a first modification example;
FIG. 11 is an operation diagram illustrating an airway expansion apparatus according to a second modification example;
FIG. 12 is a diagram illustrating a mandible holding portion and a first actuation mechanism according to a third modification example;
FIG. 13 is a diagram illustrating a second actuation mechanism according to the third modification example;
FIG. 14 is an operation diagram illustrating an airway expansion apparatus according to the third modification example;
FIG. 15 is a diagram illustrating an airway expansion apparatus according to a fourth modification example;
FIG. 16 is a view of the airway expansion apparatus according to the fourth modification example attached to the user sleeping in a supine position viewed from diagonally above;
FIG. 17 illustrates a shape change of the bag body of the airway expansion apparatus according to the fourth modification example before and after the mandible is lifted;
FIG. 18 illustrates a shape change of the bag body of the airway expansion apparatus according to the fourth modification example before and after the mandible is lifted;
FIG. 19 is an operation diagram of the airway expansion apparatus according to the fourth modification example when the user changes his/her posture;
FIG. 20 is a diagram illustrating another mode of the bag body according to the fourth modification example; and
FIG. 21 is a diagram illustrating a modification example of the pad.

### Description of Embodiments

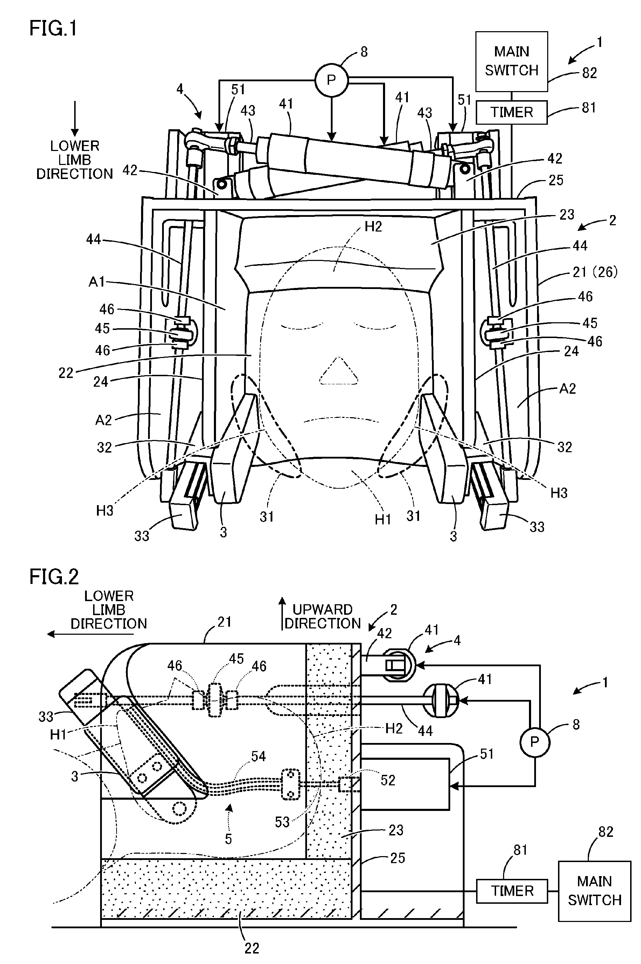

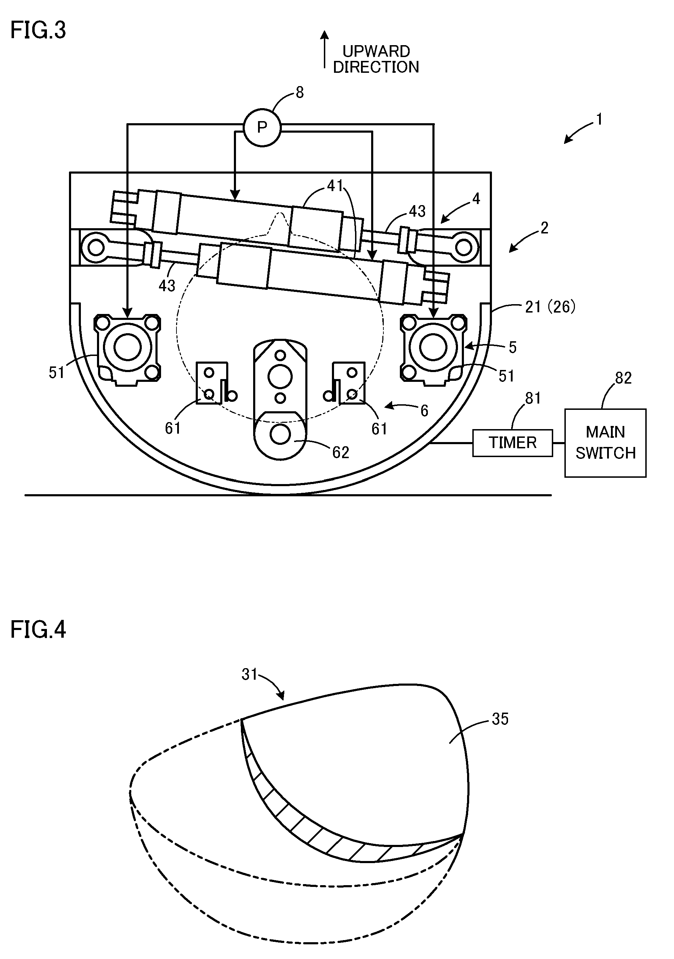

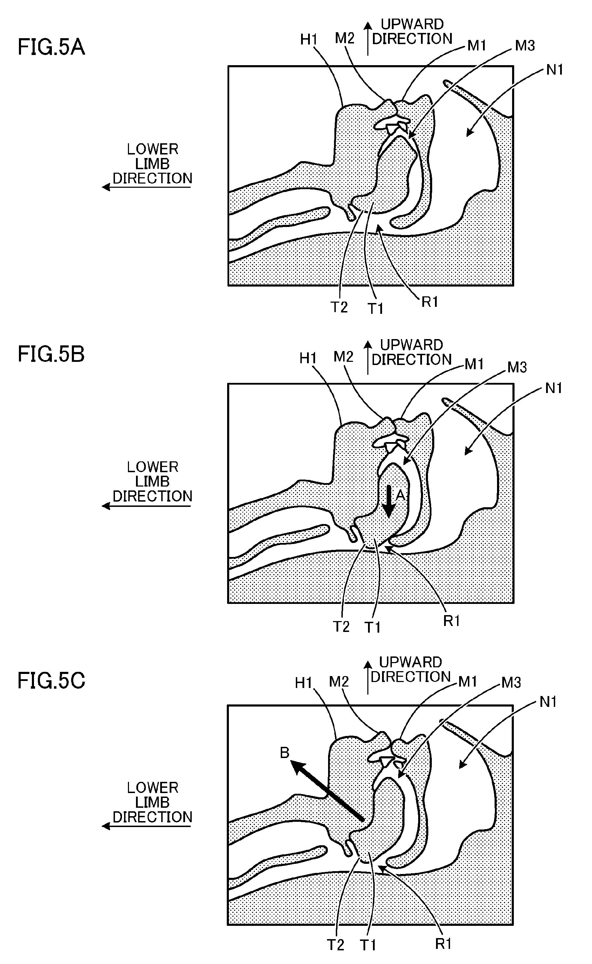

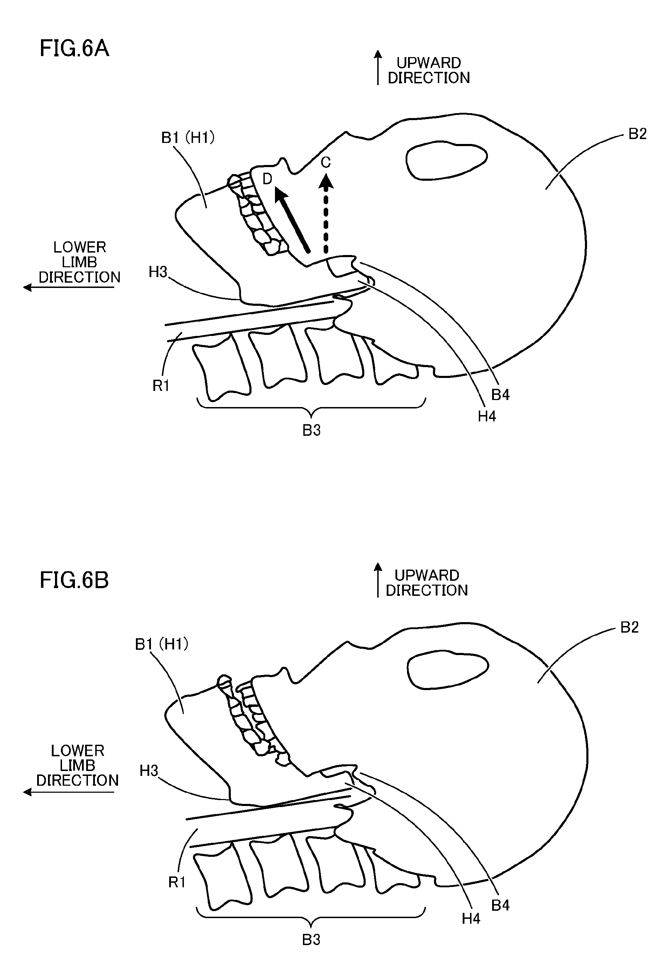

Hereinafter, an airway expansion apparatus according to embodiments of the present invention will be described with reference to FIG. 1 to FIG. 6. FIG. 1 is a top view of the airway expansion apparatus according to the present embodiment. FIG. 2 is a side view of the airway expansion apparatus according to the present embodiment. FIG. 3 is a side view of the airway expansion apparatus according to the present embodiment seen from the parietal region side. FIG. 4 is a cross-sectional perspective view of a pad according to the present embodiment. FIG. 5 is a mid-sagittal view of the tongue, oral cavity, nasal cavity and airway during sleep in a supine position. FIG. 6 is a side view of the cranial portion made up of the submaxilla, cranial bone and temporomandibular joint in a supine position with the cranial portion being supported by the pillow portion. Note that the submaxilla is a skeletal frame of a mandible H1, the temporomandibular joint is a coupling portion of the submaxilla and the cranial bone, and in the temporomandibular joint, a moving range of the mandibular condyle which is a protruding portion of the joint of the submaxilla is large.

As shown in FIG. 1, an airway expansion apparatus 1 is made up of a pillow portion 2 by which the cranial portion in a supine position is supported, provided with an airway expansion mechanism, and configured such that the airway is secured by keeping the mandible H1 held even when the lingual radix sinks. A state in which the airway is secured will be described in detail using FIG. 5. Note that suppose in the following drawings that when the user is sleeping in a supine position with the cranial portion being supported by the pillow portion, the direction in which the face is oriented is an upward direction, the direction of the lower limb viewed from the pillow portion is a lower limb direction, and the direction in which the mandible H1 is located in the upward direction and in the lower limb direction with respect to the pillow portion is a diagonally upward direction. Furthermore, suppose the direction which is the downward direction and parietal direction with respect to the face is a diagonally downward direction.

As shown in FIG. 5A, an oral cavity M3 and a nasal cavity N1 communicate with an airway R1 below a lingual radix T1. When the user is sleeping in a supine position, an upper lip M1 and a lower lip M2 contact each other and the oral cavity M3 is thereby closed. FIG. 5A illustrates a state in which the lingual radix T1 does not sink even when the user is sleeping in the supine position, a so-called state without sleep apnea. FIG. 5B illustrates a state in which when the user is sleeping in the supine position and the lingual radix T1 sinks in a direction shown by an arrow A and a sleep depth is increased, the airway R1 is blocked by the sinking lingual radix T1, thus preventing the user from breathing, that is, a state in which sleep apnea has occurred. FIG. 5C illustrates a state in which although the lingual radix T1 sinks, since the mandible H1 is lifted in the diagonally upward direction (direction shown by an arrow B), a front wall T2 of the airway R1 is also lifted and the airway R1 is kept open. The airway expansion apparatus 1 according to the present embodiment lifts the mandible H1 in the diagonally upward direction to cause the airway to expand as shown in FIG. 5C.

Furthermore, a relationship between the mandible and the airway in the supine position will be described using FIG. 6. As shown in FIG. 6A, the airway R1 exists in a space between the submaxilla B1 and the vertebra B3. In order to lift the mandible H1 in the lower limb direction and in the upward direction, the submaxilla B1 needs to be lifted in the same direction. For that purpose, an outside portion of the mandible H1 is pressed by a sufficient force so as to prevent only the skin of the mandible H1 from moving dislocated from the submaxilla B1 and the submaxilla B1 is lifted in the lower limb direction and upward direction. In that case, the mandibular condyle H4 which is the protruding portion of the joint of the submaxilla B1 is separated from the cranial bone B2 within the temporomandibular joint and lifted from the position shown in FIG. 6A to the position shown in FIG. 6B, that is, in the lower limb direction and upward direction with respect to the cranial bone B2. As a result, the frontal tissue of the airway R1 adhered to the submaxilla B1 is lifted with respect to a rear tissue of the airway R1 adhered to the vertebra B3 fixed to the cranial bone B2, causing the airway R1 to expand. When the mouth is closed or semi-open, since the articular tubercle B4 is located right above the mandibular condyle H4 in the supine position with the cranial portion being supported by the pillow portion, the mandibular condyle H4 cannot move in the upright direction shown by a dotted line arrow C. When the mouth is closed or semi-open, the mandibular condyle H4 can move only in the lower limb direction and upward direction shown by a solid line arrow D.

In FIG. 1, the pillow portion 2 is configured by attaching cushioning portions 22 and 23 to a body portion 21 which is U-figured in a side view. The body portion 21 is partitioned into a cranial portion and submaxilla accommodating space A1, and a side space A2 by a pair of left and right partition plates 24, the cushioning portions 22 and 23 are accommodated in the cranial portion and submaxillary accommodating space A1, and various mechanisms are accommodated in the side space A2. Note that the cushioning portions 22 and 23 need only to support the cranial portion, and are not particularly limited, and, for example, a low-resilient material such as low-resilient polyurethane may be used.

A top plate 25 is provided on the parietal region H2 side of the body portion 21 and a drive source such as a cylinder to drive each mechanism for airway expansion is attached to the top plate 25. A U-figured curved plate 26 of the body portion 21 is allowed to oscillate in the crosswise direction when installed on the floor and is configured to be able to easily change the posture from the supine position to the lateral position or from the lateral position to the supine position in such a way as not to prevent the user from rolling over during sleep. The pair of left and right partition plates 24 are notched at positions corresponding to the outside portion of the mandible H1 and a pair of left and right mandible holding portions 3 are provided in these parts.

The mandible holding portion 3 is formed into a rectangular block shape using a material having cushioning properties. The mandible holding portion 3 is configured to hold the outside part of the mandible H1 via a pad 31, which will be described later, on the surface. The mandible holding portion 3 is also provided with a slider 32 on an underside thereof, the slider 32 being placed on a guide rail 33 inclined diagonally upward, that is, in the direction in which the mandible H1 is pushed out from the side space A2. The proximal end of the guide rail 33 is coupled to the partition plate 24 so as to be inclinable inwardly or outwardly via a hinge portion (not shown). The mandible holding portion 3 is moved by a first actuation mechanism 4 in a direction distancing or approaching with respect to the mandible H1 and moved by a second actuation mechanism 5 in a diagonally upward/downward direction.

The first actuation mechanism 4 is configured to actuate the pair of left and right mandible holding portions 3 in a separating or approaching direction through a pair of piston cylinders 41 provided on the top plate 25. One end of each piston cylinder 41 is oscillatably supported by a bracket 42 provided on the top plate 25. A piston rod 43 protrudes from the other end of each piston cylinder 41 and one end of a connecting rod 44 is coupled to a distal end of the piston rod 43 via a ball joint. The other end of the connecting rod 44 is attached to the guide rail 33 and an intermediate portion of the connecting rod 44 is supported by a supporting portion 45 provided on the curved plate 26 via a ball joint.

An air tube of an air pump 8 which is a drive source is connected to each piston cylinder 41 and expansion/contraction of the piston rod 43 is driven by compressed air from the air pump 8. With the first actuation mechanism 4, when the piston rod 43 protrudes, the connecting rod 44 oscillates inwardly around the supporting portion 45 as an oscillating fulcrum. This causes the guide rail 33 fixed at the other end of the connecting rod 44 to incline inwardly, causing the mandible holding portion 3 to move in a direction approaching the mandible H1. On the other hand, when the piston rod 43 is pulled in, the connecting rod 44 oscillates outwardly around the supporting portion 45 as a fulcrum. This causes the guide rail 33 fixed at the other end of the connecting rod 44 to incline outwardly, causing the mandible holding portion 3 to move in a direction separating from the mandible H1.

A pair of stoppers 46 are fixed to the connecting rod 44 across the supporting portion 45 and the connecting rod 44 is configured to slide with respect to the supporting portion 45 between the pair of stoppers 46. This configuration causes the oscillating fulcrum of the connecting rod 44 to slide between the pair of stoppers 46 to thereby adjust the moving range of the mandible holding portion 3 in the distancing direction or approaching direction.

As shown in FIG. 2, the second actuation mechanism 5 is configured to actuate the pair of left and right mandible holding portions 3 in a diagonally upward/downward direction through a pair of piston cylinders 51 provided on the top plate 25. An air tube of the air pump 8 which is a drive source is connected to each piston cylinder 51 and compressed air from the air pump 8 drives expansion/contraction of a piston rod 52. The piston rod 52 protrudes into a side space A2 through an opening provided in the top plate 25 and a distal end of the piston rod 52 is coupled to the slider 32 of the mandible holding portion 3 via a flexible tube 53. The flexible tube 53 is inserted into a flexible guide tube 54 having a larger diameter than the flexible tube 53.

The flexible guide tube 54 is curved into an arcuate shape and guides the flexible tube 53 while curving it. A linear operation of the piston rod 52 in the longitudinal direction is converted to an operation in a diagonal direction via the flexible tube 53, whereby the slider 32 fixed at the distal end of the flexible tube 53 is moved along the guide rail 33. With the second actuation mechanism 5, when the piston rod 52 protrudes, the slider 32 is pushed in via the flexible tube 53. This causes the mandible holding portion 3 fixed to the slider 32 to move diagonally upward. On the other hand, when the piston rod 52 is pulled in, the slider 32 is pulled back via the flexible tube 53. This causes the mandible holding portion 3 fixed to the slider 32 to move diagonally downward.

Furthermore, the guide rail 33 is screwed to the partition plate 24 so as to make adjustable the inclination in the extending direction. In this case, since the flexible tube 53 and the flexible guide tube 54 are formed of a flexible material, the flexible tube 53 and the flexible guide tube 54 are deformed in accordance with the inclination of the guide rail 33 in the extending direction. Even when the guide rail 33 is inclined by the first actuation mechanism 4, the flexible tube 53 and the flexible guide tube 54 are deformed in accordance with the inclination operation of the guide rail 33, preventing the inclination operation of the guide rail 33 from being obstructed.

As shown in FIG. 3, the top plate 25 is provided with a switch mechanism 6 that controls a supply of compressed air to the piston cylinders 41 and 51 according to an angle of oscillation of the body portion 21. The switch mechanism 6 includes a pair of left and right switches 61 provided to cover a predetermined angle range and a pendulum portion 62 that oscillates between the pair of left and right switches 61. When the pendulum portion 62 is located between the pair of left and right switches 61, the switch mechanism 6 performs control so as to push out the piston cylinders 41 and 51 and pull in the piston cylinders 41 and 51 by the pendulum portion 62 contacting any one of the pair of left and right switches 61. Note that details of operation of the switch mechanism 6 will be described later.

A timer 81 that delays the operation of the second actuation mechanism 5 with respect to the first actuation mechanism 4 and a main switch 82 that turns ON/OFF the power of the airway expansion apparatus 1 are connected to the airway expansion apparatus 1. The timer 81 operates so as to delay the timing of a compressed air supply to the piston cylinder 51 of the second actuation mechanism 5 with respect to the timing of a compressed air supply to the piston cylinder 41 of the first actuation mechanism 4. Thus, it is possible to actuate the second actuation mechanism 5 after actuating the first actuation mechanism 4 using the single air pump 8.

Furthermore, with the airway expansion apparatus 1, the pads 31 are attached to the pair of left and right mandible holding portions 3 to hold the outside part of the mandible H1. As shown in FIG. 4, the pad 31 is formed of a cushioning material such as silicone into a bowl-like shape having a semilunar cross section so as to come into close contact with the outside part of the mandible H1. The pad 31 having such a shape is deformed so as to follow the shape of the outside part of the mandible H1 to increase adherence to the outside part of the mandible H1 (see FIG. 1). Moreover, a contact surface 35 of the pad 31 has adhesiveness, which prevents dislocation with respect to the outside part of the mandible H1. The contact surface 35 of the pad 31 is curved so as to follow the mandibular angle H3, the curvature of the pad 31 is caught in the mandibular angle H3 and the level of unity between the outside part of the mandible H1 and the mandible holding portion 3 is raised via the pad 31. In this way, the outside part of the mandible H1 is held to the mandible holding portion 3 via the pad 31, and it is thereby possible to prevent dislocation of the mandible with respect to the mandible holding portion 3.

With the airway expansion apparatus 1 configured in this way, when the first actuation mechanism 4 is driven, the mandible H1 is thereby sandwiched between the pair of left and right mandible holding portions 3 and the second actuation mechanism 5 is driven in this condition, and the pair of left and right mandible holding portions 3 are thereby kept lifted in an diagonally upward direction. For this reason, the mandible H1 in the supine position is also pushed out in the diagonally upward direction and the front wall T2 of the airway R1 is also kept lifted upward accordingly. Thus, even when the lingual radix T1 sinks, the airway R1 is still secured, making it possible to prevent a sleep apnea syndrome. Furthermore, since the curved plate 26 of the pillow portion 2 is formed into a U-figured shape, it is possible to cancel the holding by the mandible holding portions 3 in the lateral position through the switch mechanism 6 without preventing roll over during sleep.

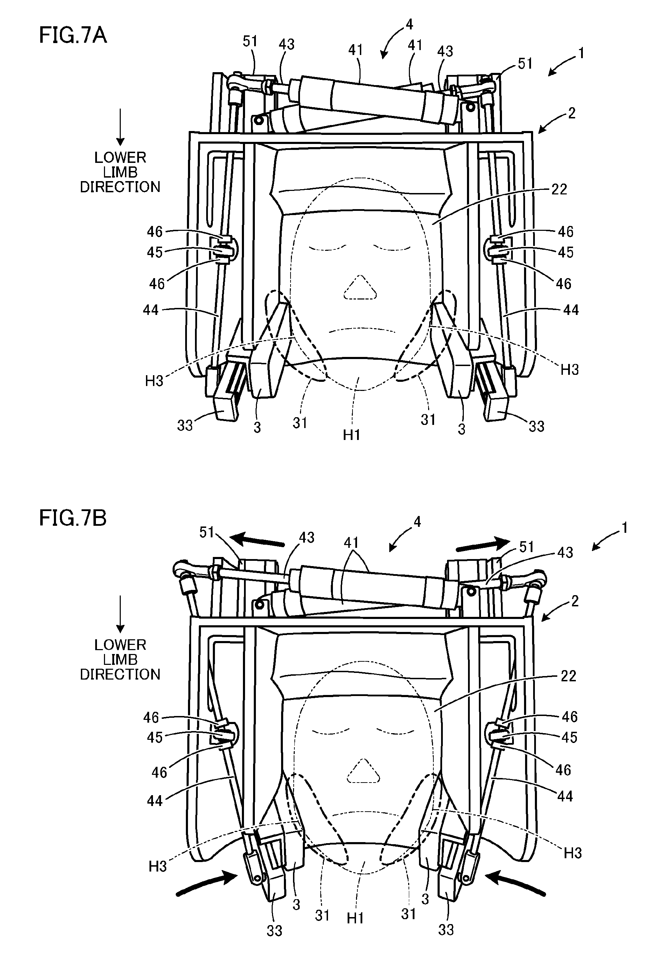

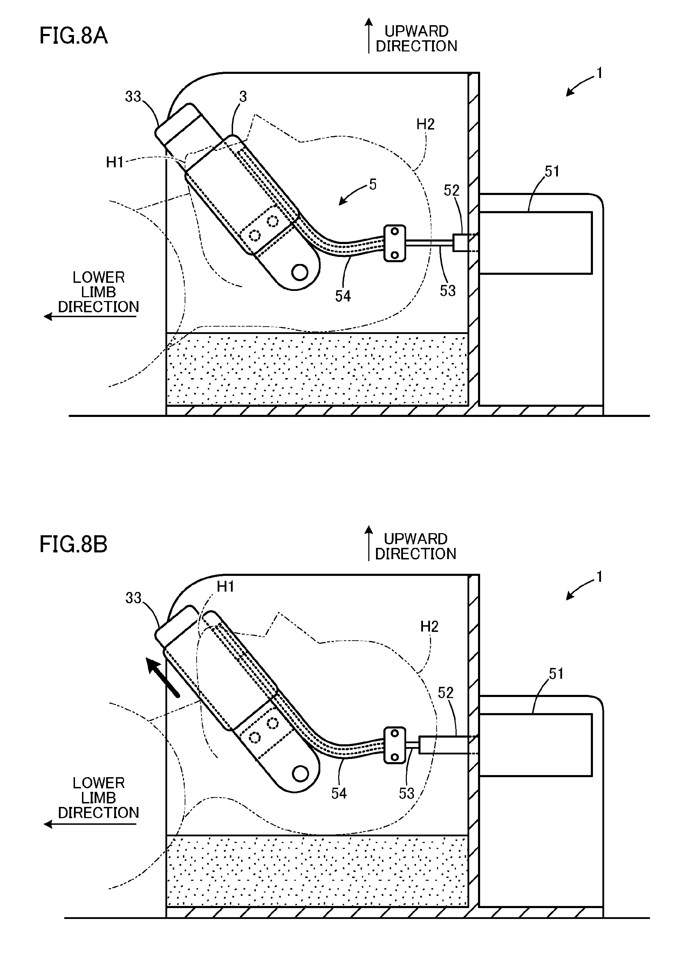

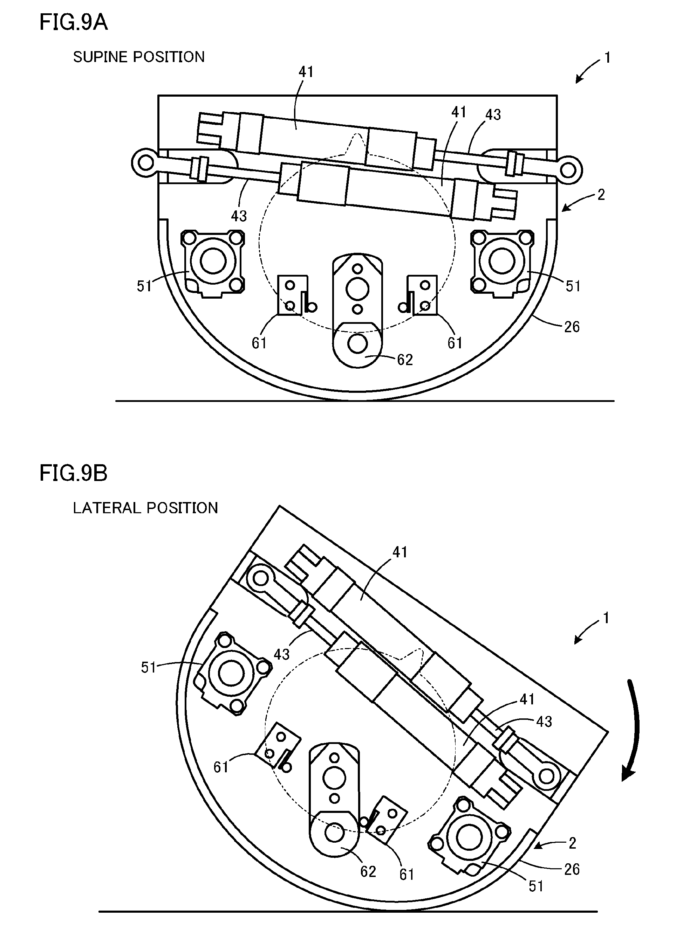

Next, operation of the airway expansion apparatus 1 will be described in detail with reference to FIG. 7 to FIG. 9. FIG. 7 is an operation diagram illustrating a mandible holding operation of the airway expansion apparatus according to the present embodiment. FIG. 8 is an operation diagram illustrating a mandible lifting operation of the airway expansion apparatus according to the present embodiment. FIG. 9 is an operation diagram illustrating a switching operation of the switch mechanism of the airway expansion apparatus according to the present embodiment. In FIG. 8, only the pillow portion and the second actuation mechanism are described and descriptions of the other parts are omitted for convenience of description.

First, as shown in FIG. 7A, the user attaches the pad 31 to both the outside parts of the mandible H1 and places the cranial portion on the cushioning portion 22. This causes the cranial portion to sink into the cushioning portion 22 appropriately and the pair of mandible holding portions 3 are located on both sides of the mandible H1. In this case, the distance between the pair of stoppers 46 of the left and right connecting rods 44 and the inclination of the guide rail 33 in the extending direction are adjusted in advance according to the size and shape of the user's face. If the main switch 82 is turned on in this condition, compressed air is supplied to the pair of piston cylinders 41 from the air pump 8 and operation of the first actuation mechanism 4 starts (see FIG. 1).

Next, as shown in FIG. 7B, when compressed air is supplied to each piston cylinder 41, the piston rod 43 of each piston cylinder 41 is pushed outward. The connecting rod 44 coupled to the distal end of the piston rod 43 is oscillated inwardly around the supporting portion 45 as an oscillation fulcrum and the guide rail 33 fixed to connecting rod 44 is inclined inwardly. This causes the mandible holding portion 3 to move in a direction approaching the mandible H1 and causes the mandible holding portion 3 to come into contact with the outside part of the user's mandible H1 via the pad 31. The pair of left and right mandible holding portions 3 come into contact with the outside parts of the user's mandible H1 from the left and right, and the outside parts of the mandible H1 are thereby held so as to be integrated with the mandible holding portions 3 via the pair of pads 31. In this case, the mandible holding portions 3 are pressed moderately by the first actuation mechanism 4 and the outside parts of the mandible H1 are pressed by the mandible holding portions 3.

As described above, the pad 31 has a shape that follows the outside part of the mandible H1, and further has the contact surface 35 (see FIG. 4) that is curved so as to follow the mandibular angle H3. For this reason, when the outside parts of the mandible H1 are sandwiched between the pair of mandible holding portions 3 via the pads 31, unity between the pair of mandible holding portions 3 and the outside parts of the mandible H1 is improved. Since the contact surface 35 of the pad 31 has adhesiveness, dislocation of the outside part of the mandible H1 with respect to the contact surface 35 of the pad 31 can be prevented and the pad 31 is less likely to be separated from the outside part of the mandible H1.

Next, as shown in FIG. 8A, when a set time of the timer 81 (see FIG. 1) elapses, compressed air is supplied to the piston cylinder 51 as well and the second actuation mechanism 5 starts to operate. In this initial state, the piston rod 52 is drawn in by the piston cylinder 51 and the pair of left and right mandible holding portions 3 are located at the bottom end of the guide rails 33 while holding the outside parts of the mandible H1.

Next, as shown in FIG. 8B, when compressed air is supplied to each piston cylinder 51, the piston rod 52 of each piston cylinder 51 is pushed out in a direction from the parietal region H2 toward the mandible H1. The flexible tube 53 attached to the piston rod 52 is pushed out by being guided by the flexible guide tube 54 and the mandible holding portion 3 fixed to the flexible tube 53 is moved upward along the guide rail 33. The guide rail 33 is inclined so as to elevate from the mandibular angle H3 toward the mandible, and the pair of mandible holding portions 3 are pushed up integrally with the outside parts of the mandible H1 along the guide rails 33. That is, since the mandible holding portions 3 are moderately pressed by the first actuation mechanism 4, the whole mandible H1 is lifted and moved without only the skin of the outside parts of the mandible H1 moving dislocated from the submaxilla. Thus, the mandible H1 is kept lifted diagonally upward, and the front wall of the airway is also kept lifted upward accordingly. Therefore, even when the lingual radix sinks, the airway is still secured, thus preventing a sleep apnea syndrome from appearing. Thus, the airway expansion apparatus 1 is configured so as not to cause the entire cranial portion to rotate backward with respect to the cervical vertebra like a head tilt method but to lift the mandible in a diagonally upward direction.

Next, as shown in FIG. 9A, when the user is sleeping in a supine position, the pendulum portion 62 is located between the pair of switches 61, that is, within a predetermined angle range. In this condition, compressed air continues to be supplied to the piston cylinders 41 and 51, and the operating states of the first and second actuation mechanisms 4 and 5 are kept whereby the mandible is held in a diagonally upward direction by the pair of mandible holding portions 3.

Next, as shown in FIG. 9B, when the user changes the posture from the supine position to the lateral position, the U-figured curved plate 26 of the pillow portion 2 oscillates with respect to the floor surface. The pendulum portion 62 oscillates up to outside a predetermined angle range relative to the pillow portion 2 and comes into contact with any one of the pair of left and right switches 61. When the switch 61 contacts the pendulum portion 62, the supply of compressed air to the piston cylinders 41 and 51 is controlled, the piston rod 43 of the piston cylinder 41 is pulled back and the piston rod 52 (see FIG. 8) of the piston cylinder 51 is pulled back. This causes the mandible holding portions 3 to return to the initial position and the state is changed to a released state in which the operations of the first and second actuation mechanisms 4 and 5 are canceled, that is, the mandible holding portion is not lifted in a diagonally upward direction.

Furthermore, when the user changes his/her posture from a lateral position to a supine position, the first and second actuation mechanisms 4 and 5 are actuated again and the mandible is held in the above-described order. Thus, the lifting of the mandible H1 by the mandible holding portions 3 in the lateral position is canceled so that the mandible H1 can be lifted only in the supine position in which sleep apnea may occur. Furthermore, by adjusting a predetermined angle range in accordance with a postural change between the supine position and the lateral position, it is possible to lift the mandible only in a supine position or a posture similar to a supine position in which sleep apnea may occur.

As described above, according to the airway expansion apparatus 1 according to the present embodiment, when the user places his/her cranial portion on the pillow portion 2, the mandible H1 is integrally held by the pair of left and right mandible holding portions 3 and the mandible H1 is kept lifted. Thus, it is possible to prevent a sleep apnea syndrome and avoid a burdensome operation of bringing one end of the airway expansion apparatus 1 into close contact with the cranial portion and reliably fix it in advance before the user going to sleep. Moreover, in this configuration only the submaxilla is held and the cranial portion is not fastened, and it is therefore possible to obtain a comfortable sleep free of a sense of oppression.

Note that the present invention is not limited to the above-described embodiment, but can be implemented modified in various ways. The size and shape of the above-described embodiment are not limited to those illustrated in the accompanying drawings, but can be changed as appropriate within a range in which effects of the present invention are exhibited. In addition, the present invention can be implemented modified as appropriate without departing from the scope of the object of the present invention.

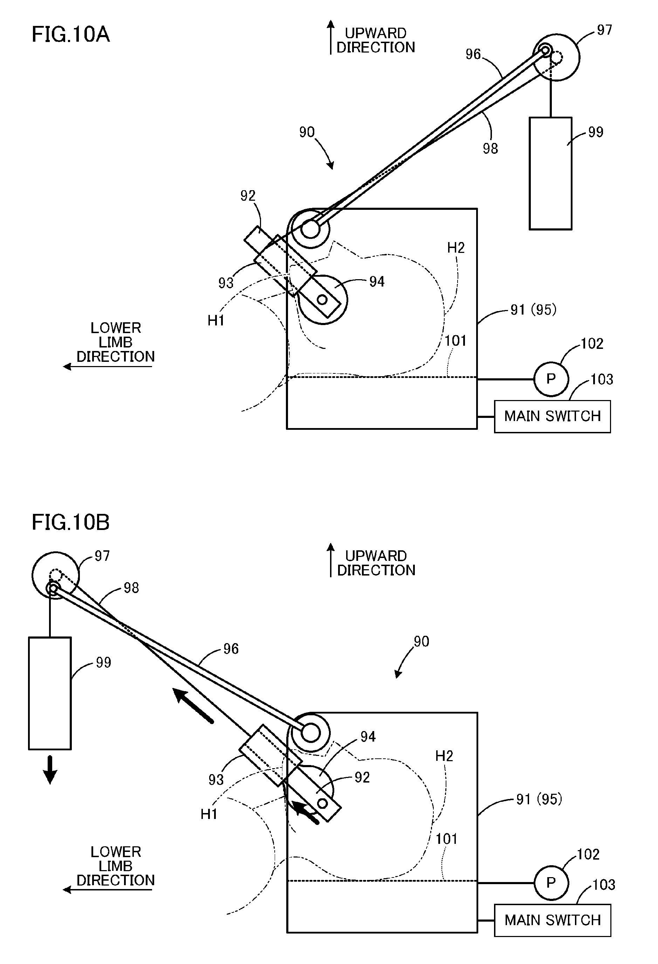

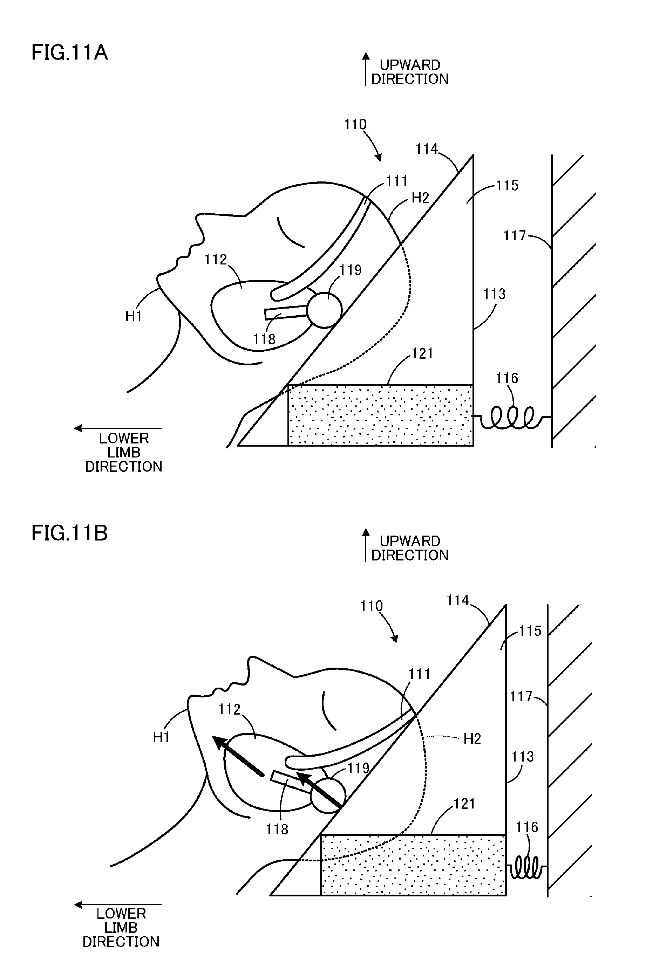

For example, modification examples shown in FIG. 10 and FIG. 11 may also be adopted. A first modification example will be described in brief with reference to FIG. 10. The first modification example is different from the present embodiment in that the weight of a weight portion is used as the drive source of the second actuation mechanism instead of the air pump. FIG. 10 is an operation diagram illustrating an airway expansion apparatus according to the first modification example.

As shown in FIG. 10, with an airway expansion apparatus 90 according to the first modification example, a guide rail 92 extending diagonally upward is set up in a pillow portion 91 and a slider 93 is slidably attached to the guide rail 92. The slider 93 is provided with a bag body 94 that inflates with compressed air, and as the bag body 94 inflates, the mandible H1 is held via the pad 31 (see FIG. 4). That is, in the first modification example, the bag body 94 functions as a mandible holding portion. A proximal end side of an arm 96 is oscillatably supported by a body portion 95 of the pillow portion 91. A pulley 97 is fixed on a distal end side of the arm 96. A metal wire 98 extends to this pulley 97, the slider 93 is fixed to one end of the metal wire 98 and a weight portion 99 is fixed at the other end of the metal wire 98.

As shown in FIG. 10A, when using the airway expansion apparatus 90 according to the first modification example, the user turns the arm 96 and places the weight portion 99 on the parietal region side. In this way, when the cranial portion is placed on the cushioning portion 101, the weight portion 99 is kept out of the way. Next, when the user attaches the pads 31 on both sides of the mandible H1 and places his/her cranial portion on a cushioning portion 101, the cranial portion sinks appropriately into the cushioning portion 101 and the pair of bag bodies 94 are placed on both sides of the mandible H1. When a main switch 103 is turned on in this condition, compressed air is supplied to the pair of piston cylinders 41 from an air pump 102, the bag bodies 94 inflate and the bag bodies 94 come into contact with the user's mandible H1 via the pads 31.

With the pair of left and right bag bodies 94 contacting the outside parts of the user's mandible H1 from the left and right, the mandible H1 is held via the pair of pads 31 so as to move integrally with the mandible holding portions 3. That is, in the first modification example, the air pump 102 that supplies compressed air to the bag bodies 94 functions as the first actuation mechanism. In this case, since the slider 93 is pulled in a direction substantially perpendicular to the guide rail 92 by gravity of the weight portion 99, the bag bodies 94 fixed to the slider 93 never move upward.

Next, as shown in FIG. 10B, the user turns the arm 96 and places the weight portion 99 above the abdomen. In this way, the gravity of the weight portion 99 is converted by the pulley 97 to a direction in which the slider 93 is lifted, and the slider 93 fixed to the metal wire 98 is moved upward along the guide rail 92 by the gravity of the weight portion 99. Thus, the pair of bag bodies 94 are lifted while holding the outside parts of the mandible H1 and the mandible H1 is kept lifted. Thus, by converting the gravity of the weight portion 99 using a power conversion mechanism made up of the pulley 97 and the metal wire 98, it is possible to keep the mandible H1 lifted without using any drive source such as an air pump in a low-cost configuration.

A second modification example will be described briefly with reference to FIG. 11. The second modification example is different from the present embodiment in that the weight of the cranial portion is used as a drive source of the actuation mechanism instead of the air pump. FIG. 11 is an operation diagram of an airway expansion apparatus according to the second modification example.

As shown in FIG. 11, with an airway expansion apparatus 110 according to the second modification example, a pair of left and right mandible holding portions 112 are attached at both ends of an expandable resin band 111, and by attaching the resin band 111 to the cranial portion, the outside parts of the mandible H1 are sandwiched between the pair of left and right mandible holding portions 112. A pillow portion 113 is provided with a pair of left and right side plates 115 on which a slope 114 that elevates from the mandible H1 toward the parietal region H2 in correspondence with the mandible holding portions 112 is formed. Each side plate 115 is connected to a wall 117 via an urging member 116. An arm 118 extends from each mandible holding portion 112 toward an occipital region side and a roller-shaped abutting portion 119 is rotatably attached at a distal end of the arm 118.

As shown in FIG. 11A, when using the airway expansion apparatus 110 according to the second modification example, the user attaches the resin band 111 on the cranial portion in such a way that the outside parts of the mandible H1 are sandwiched between the pair of left and right mandible holding portions 112 by an elastic force of the resin band 111. In this way, the outside parts of the mandible H1 are held integrally with the mandible holding portions 112. Next, when the user places the cranial portion on the cushioning portion 121, the abutting portion 119 abuts on the slopes 114 of the pair of left and right side plates 115.

As shown in FIG. 11B, when the cranial portion sinks into the cushioning portion 121, the abutting portions 119 move downward along the slopes 114 of the pair of left and right side plates 115. As the abutting portions 119 move, the mandible holding portions 112 are pushed out in a direction perpendicular to the slopes 114 and the mandible H1 is kept lifted by the mandible holding portions 112. Thus, the mandible holding portions 112 can be lifted using the weight of the cranial portion and the mandible H1 can be kept lifted in a low-cost configuration without using any drive source such as an air pump.

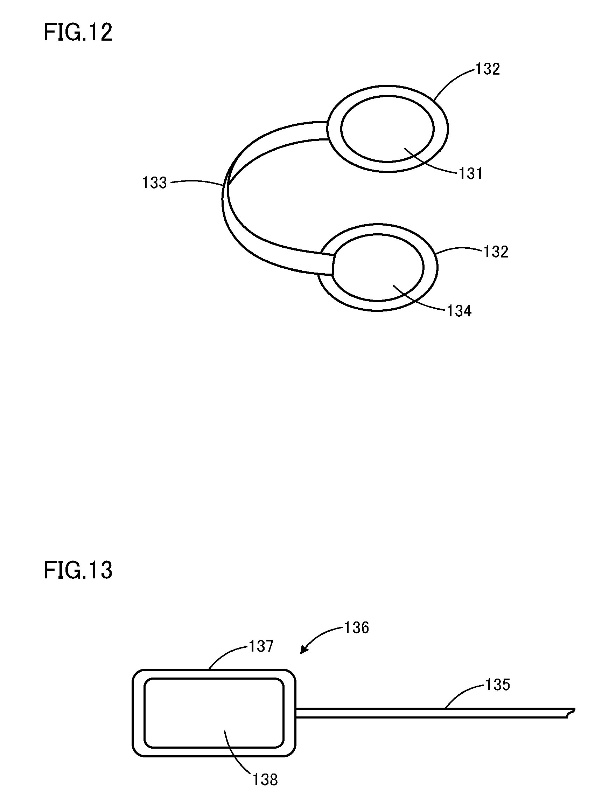

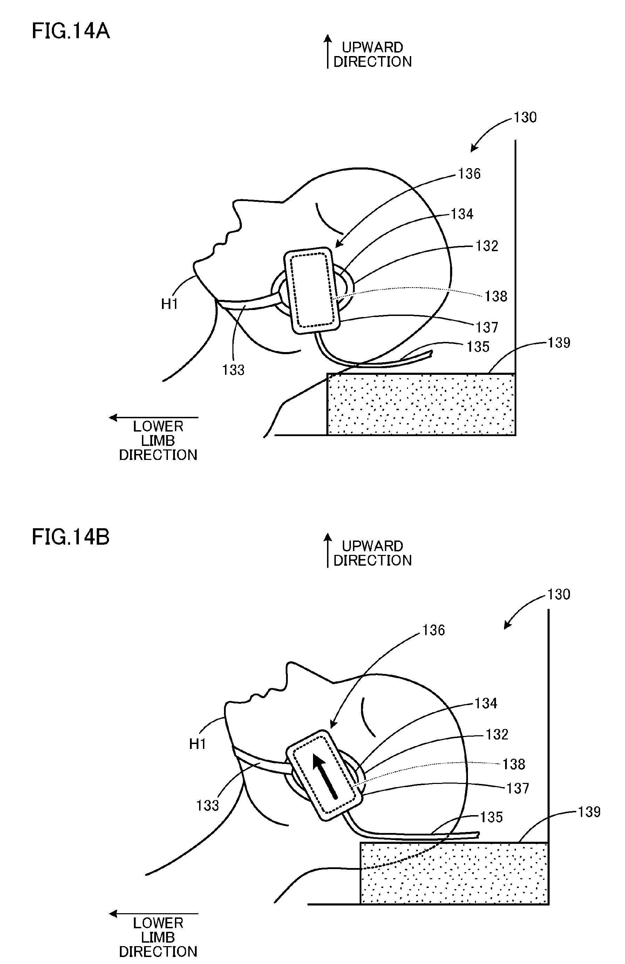

Furthermore, for example, modification examples as shown in FIG. 12 to FIG. 14 may also be adopted. A third modification example will be described with reference to FIG. 12 to FIG. 14. FIG. 12 is a diagram illustrating mandible holding portions and a first actuation mechanism according to the third modification example. FIG. 13 is a diagram illustrating a second actuation mechanism according to the third modification example. FIG. 14 is an operation diagram illustrating the airway expansion apparatus according to the third modification example. Note that in FIG. 13 and FIG. 14, only a part of the second actuation mechanism or cushioning portion will be described and description of the other parts will be omitted, for convenience of description.

In the above-described embodiment, the mandible holding portions that hold the mandible via pads that comes into close contact with the mandible and the first actuation mechanism are undetachably assembled into the airway expansion apparatus. In contrast, the third modification example provides a configuration in which the mandible holding portions and the first actuation mechanism are detachable from the second actuation mechanism.

As shown in FIG. 12, a pair of left and right mandible holding portions 132 are attached at both ends of an elastic U-figured resin band 133. Pads 131 are adhered to the surfaces of the mandible holding portions 132 to be pasted to the mandible H1 and magic tapes (registered trademark) 134 are adhered to the opposite surfaces. Since the U-figured resin band 133 presses the pair of left and right mandible holding portions 132 against the mandible H1 by an elastic force thereof, the resin band 133 operates as the first actuation mechanism.

Furthermore, as shown in FIG. 13, an actuator 137 of a second actuation mechanism 136 is provided at a distal end of a flexible tube 135 that makes up the second actuation mechanism 136 and a magic tape (registered trademark) 138 is adhered to one side thereof. The magic tape 138 is detachable from the magic tape 134 pasted to the mandible holding portion 132 and by removing the magic tape 134 from the magic tape 138, the first actuation mechanism can be removed from the second actuation mechanism 136.

As shown in FIG. 14A, when using the airway expansion apparatus 130 according to the third modification example, the user attaches the U-figured resin band 133 so as to be fitted to the mandible before going to sleep, and then presses the outside parts of the mandible H1 via the pad 131 attached to the pair of left and right mandible holding portions 132 attached at both ends of the resin band 133. Thus, the outside parts of the mandible H1 are held so as to be integrated with the mandible holding portions 132 via the pads 131.

Next, as shown in FIG. 14B, when the user places his/her cranial portion on a cushioning portion 139 and the cranial portion sinks into the cushioning portion 139, the user manually connects the magic tapes 138 adhered to the actuator 137 of the second actuation mechanism 136 with the magic tapes 134 pasted to the mandible holding portions 132. Thus, when the user is sleeping in a supine position, the mandible H1 is kept lifted diagonally upward and the front wall of the airway is also kept lifted upward. Therefore, even when the lingual radix falls, the airway is secured, thus preventing a sleep apnea syndrome.

In this way, the mandible holding portions 132 and the resin band 133 are attached to the outside parts of the mandible H1 in advance before the user going to sleep, and when the user goes to sleep, the resin band 133 and the second actuation mechanism 136 are connected together via the mandible holding portions 132, and the second actuation mechanism 136 operates so as to lift the mandible holding portions 132. As described above, it is possible to make the configuration of the airway expansion apparatus 130 more simple. Note that although the first actuation mechanism is configured of the resin band 133 in the third modification example, the first actuation mechanism may also be configured to mechanically actuate the mandible holding portions 132 so as to come into contact with the outside parts of the mandible H1.

In the above-described embodiment, the first actuation mechanism 4 is configured to be actuated by an air cylinder, but the present invention is not limited to this configuration. The first actuation mechanism 4 may be configured to actuate the mandible holding portions 3 so as to come into contact with the outside parts of the mandible H1, and may be configured to be actuated by, for example, an electric actuator.

In the above-described embodiment, the second actuation mechanisms 5 and 136 are configured to be actuated by an air cylinder, but the present invention is not limited to this configuration. The second actuation mechanisms 5 and 136 need only to actuate the mandible holding portions 3 and 132 so as to be lifted with respect to the pillow portion 2 or cushioning portion 139, and may be configured to be actuated by, for example, an electric actuator.

In the above-described embodiment, the pair of left and right mandible holding portions 3 and the pads 31 are configured as separate bodies, but the present invention is not limited to this configuration. The pair of left and right mandible holding portions 3 and the pads 31 may be formed into a single unit.

In the above-described embodiment, the switch mechanism 6 is configured of the pair of switches 61 and the pendulum portion 62, but the present invention is not limited to this configuration. The switch mechanism 6 needs only to be configured to switch between an operating state and a released state of the first and second actuation mechanisms 4 and 5 according to an angle of oscillation of the pillow portion 2 (body portion 21). For example, an angle sensor may be used as the switch mechanism 6. The switch mechanism 6 may have any configuration if it is at least possible to switch between the operating state and the released state of the second actuation mechanism 5.

### (Evaluation Experiment)

Ten object people (all men) were asked to wear the airway expansion apparatus 1 according to the present embodiment shown in FIG. 1, bite rubber before and after wearing the airway expansion apparatus 1 and a distance between an upper front tooth and a lower front tooth before and after wearing the airway expansion apparatus 1 was measured respectively based on toothprints remaining in the rubber. A horizontal moving distance of the mandible was calculated from the distance between the upper front tooth and the lower front tooth before the wearing of the apparatus and the distance between the upper front tooth and the lower front tooth after the wearing of the apparatus. That is, if it is assumed that the distance between the upper front tooth and the lower front tooth before the wearing of the apparatus is L1 and the distance between the upper front tooth and the lower front tooth after the wearing of the apparatus is L2, (L1-L2) is the moving distance of the mandible by the wearing of the apparatus. According to this experiment, an average horizontal moving distance with the ten object people was 3.7 mm. Table 1 shows the experiment results.

### [Table 1]

### Subject person

### Difference (mm)

### Average

### Standard deviation

According to the following reference, it is possible to prevent a sleep apnea syndrome if the horizontal moving distance of the mandible is equal to or greater than 3.5 mm, and therefore the apparatus of the present invention is effective in preventing a sleep apnea syndrome (reference (Kazuhisa Ezaki: Clarification of Effect Expression Mechanism of Separate Adjustment Type Splint Therapy for Sleep Apnea Syndrome, Research Project Number: 08771923. Principal Investigator. FY1996. Researcher Number: 80203628. Grants-In-Aid for Scientific Research Database National Institute of Informatics)).

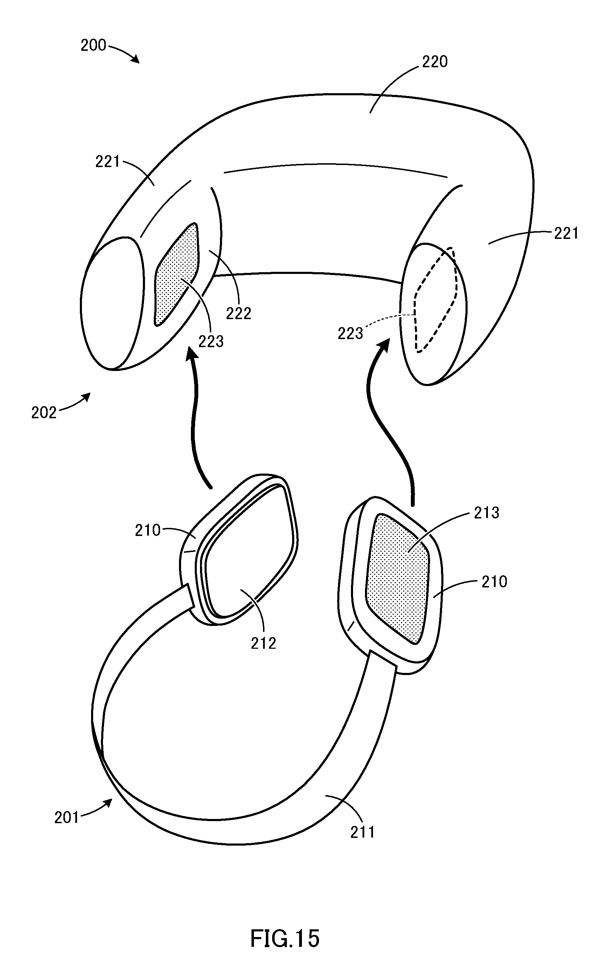

Since the configuration of the apparatus of the above-described embodiment is complicated and large, it is not easy to carry the apparatus during a travel or the like. Thus, the applicant of the present application has discovered a method for lifting the mandible by adopting a configuration including a bag body whose interior can be filled with a fluid such as air and a mandible holding unit that holds the submaxilla and by taking advantage of the fact that when the cranial portion is placed on the bag body, the fluid in the bag body flows under the own weight of the cranial portion and the bag body is thereby deformed. This simplifies the configuration of the airway expansion apparatus and allows it to be carried about. Hereinafter a fourth modification example will be described with reference to FIG. 15 to FIG. 20.

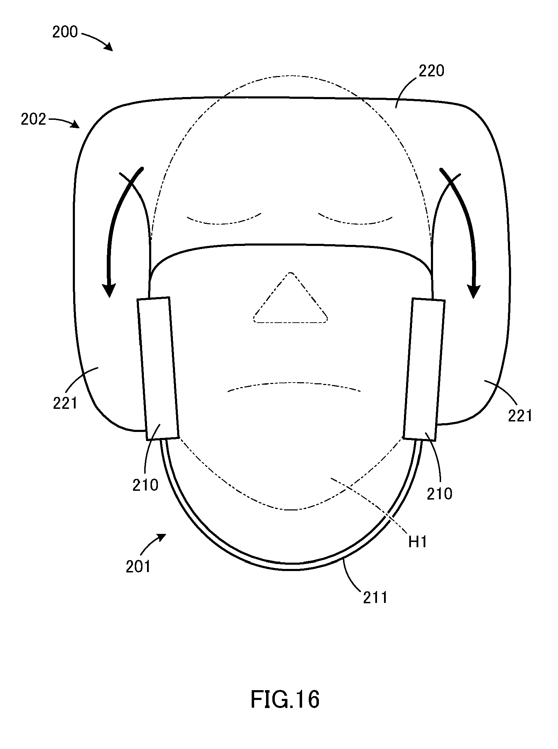

First, a schematic configuration of the airway expansion apparatus according to the fourth modification example will be described with reference to FIG. 15 and FIG. 16. FIG. 15 is a diagram illustrating the airway expansion apparatus according to the fourth modification example. FIG. 16 shows a top view seen from diagonally above when the user wearing the airway expansion apparatus according to the fourth modification example is sleeping in a supine position.

As shown in FIG. 15, an airway expansion apparatus 200 according to the fourth modification example is provided with a mandible holding unit 201 that holds the submaxilla and a bag body 202 that is coupled to the mandible holding unit 201 and filled with a fluid such as air. With this airway expansion apparatus 200, when the cranial portion is placed on the bag body 202, the bag body 202 is crushed under the own weight of the cranial portion, the fluid contained in the bag body 202 flows, causing the bag body 202 to deform. The deformation of the bag body 202 causes the mandible holding unit 201 coupled to the bag body 202 to move. When the mandible holding unit 201 moves, the mandible held to the mandible holding unit 201 is lifted relative to the cranial portion, allowing the airway to expand.

The mandible holding unit 201 plays the role of firmly fixing the mandible so as to allow the mandible to move relative to the cranial portion. The mandible holding unit 201 is provided with a pair of mandible holding portions 210 that hold the mandible coupled to the cranial portion at the temporomandibular joint and a coupling portion 211 that couples the pair of mandible holding portions 210 and keeps the mandible holding portion in contact with the mandible. In the configuration shown in FIG. 15, the pair of left and right rectangular mandible holding portions 210 are coupled by the U-figured band portion 211 (coupling portion). A pad portion 212 that comes into contact with the mandible is provided on one surface (face side) of each mandible holding portion 210 and a magic tape 213 (registered trademark) coupled to the bag body 202 is provided on the other surface (outside). The pair of left and right mandible holding portions 210 are connected to the band portion 211 with one side provided with the pad portions 212 facing the other side.

The band portion 211 has elasticity. For this reason, when the mandible holding unit 201 is attached to the mandible, the pad portion 212 comes into contact with the mandible (to be more specific, an edge of the submaxilla), the mandible is sandwiched and held between the pair of left and right mandible holding portions 210. In this case, an urging force acts on the band portion 211 in directions in which the pad portions 212 face and approach each other. Thus, the band portion 211 plays the role of keeping the mandible holding portions 210 (pad portions 212) in contact with the mandible. Moreover, since the surface of the pad portion 212 has adhesiveness, it is possible to keep the pad portion 212 in close contact with the mandible. This prevents the mandible holding portion 210 from being dislocated from the mandible.

The bag body 202 functions as a pillow to support the cranial portion during sleep and also functions as an actuation section that is coupled to the mandible holding unit 201 to actuate the mandible holding portion 210 so as to be lifted upward. The bag body 202 is formed of a soft material such as vinyl or cloth and the bag body 202 is provided with an air inlet (not shown) to allow a fluid (a case will be described here where the fluid is air) such as air to be freely charged into/discharged from the bag body 202. When the bag body 202 is not in use, the bag body 202 can be collapsed into a compact size by removing air from the bag body 202. This improves convenience when the apparatus is carried about during a travel or the like.

When the bag body 202 is used, the bag body 202 is filled with air to such an extent that the bag body 202 is not completely inflated so that the interior of the bag body 202 has certain looseness. That is, the extent that the bag body 202 is not completely inflated means that the volume of the air filling the inside of the bag body 202 is smaller than the volume (capacity) of the bag body 202. The ratio of the volume of the air to the volume (capacity) of the bag body 202 may not particularly be limited if it is to such an extent that when the cranial portion is placed on the bag body 202, the air in the bag body 202 is caused to move under the own weight of the cranial portion, the bag body 202 is deformed, and this deformation causes the mandible holding unit 201 to move, lifting the mandible relative to the cranial portion and thereby achieving an effect of the present invention of allowing the airway to expand.

The bag body 202 filled with air has a substantially U-figured shape so as to cover around the user's neck and is constructed of a cranium supporting portion 220 that extends in a left-right direction and supports the cranial portion and a pair of protruding portions 221 that extend from both ends of the cranium supporting portion 220 and are coupled to the pair of mandible holding portions 210 respectively. The cranium supporting portion 220 and the pair of protruding portions 221 are configured to be filled with air respectively, the interior of the cranium supporting portion 220 and the interior of the pair of protruding portions 221 communicate with each other, thereby allowing the air to flow. The bag body 202 is attached so as to cover around the user's neck (cervical vertebra) through the cranium supporting portion 220 and the pair of protruding portions 221. Here, the volume of the region of the bag body 202 attached to the mandible holding portion 210 of the mandible holding unit 201 (region corresponding to the protruding portion 221) is preferably smaller than the volume of the region of the bag body 202 that supports the cranial portion (region corresponding to cranium supporting portion 220). Thus, when the cranial portion is placed on the bag body 202, the air in the bag body 202 is moved under the own weight of the cranial portion causing the bag body 202 to deform, and this deformation causes the mandible holding unit 201 to move, thus making it possible to effectively realize the operation of lifting the mandible relative to the cranial portion.

The length of the cranium supporting portion 220 in the left-right direction is such a length that supports the user's cranial portion and allows the user to roll over during sleep. The thickness of the cranium supporting portion 220 in the front-back direction (direction orthogonal to the left-right direction in which the cranium supporting portion 220 extends) is formed to be larger than the thickness of the pair of protruding portions 221 in the left-right direction (direction orthogonal to the direction in which the protruding portion 221 protrudes). Furthermore, the thickness of the cranium supporting portion 220 in the height direction is such that cushioning properties are kept even when the cranium supporting portion 220 is crushed under the own weight of the cranial portion. Thus, even when the cranium supporting portion 220 is crushed when the cranial portion is placed thereon, the cranium supporting portion 220 has a volume enough to stably support the cranial portion so as to be able to keep cushioning properties.

On the other hand, the protruding portion 221 protrudes so as to follow the user's mandible and has such a length that it comes into contact with the edge of the submaxilla. Each protruding portion 221 is formed so as to have a smaller volume than that of the cranium supporting portion 220. Although details will be described later, the protruding portion 221 has such a volume that as a result of inflating with the air flowing from the cranium supporting portion 220, the protruding portion 221 becomes tensioned and lifted upward. As described above, since a smaller volume of air than the volume (capacity) of the bag body 202 is charged into the bag body 202, when any given part of the bag body 202 is crushed, it is possible to cause the air in the bag body 202 to freely flow inside the bag body 202.

For example, when the cranial portion is placed on the cranium supporting portion 220, the cranium supporting portion 220 is crushed under the own weight of the cranial portion, the air in the cranium supporting portion 220 flows toward the protruding portions 221 at both ends. As a result, each slackened protruding portion 221 is inflated with the air flown from the cranium supporting portion 220. On the other hand, when the protruding portion 221 is crushed, the air in the protruding portion 221 flows toward the cranium supporting portion 220. In this way, the air in the bag body 202 is allowed to flow between the cranium supporting portion 220 and each protruding portion 221.

Rectangular magic tapes 223 are provided on respective inside surfaces 222 facing each other on distal end sides of the pair of protruding portions 221. The magic tapes 213 of the mandible holding unit 201 are attached to the magic tapes 223. In this way, the bag body 202 is detachable from the mandible holding unit 201. Therefore, it is possible to attach the mandible holding unit 201 to the mandible in advance before the user going to sleep, attach the bag body 202 so as to wind around the neck, and couple the mandible holding unit 201 with the bag body 202 via the magic tapes 213 and 223. Thus, since the mandible holding unit 201 and the bag body 202 can be attached separately, it is possible to align the mandible holding portion 210 with the mandible and align the bag body with the cranial portion separately. Therefore, it is possible to simplify a burdensome operation of attaching the airway expansion apparatus 200.

As described above, the airway expansion apparatus 200 according to the fourth modification example is constructed of only the mandible holding unit 201 and the bag body 202, and it is thereby possible to simplify the configuration. When using the airway expansion apparatus 200, the mandible holding unit 201 is attached to the mandible H1 before the user going to sleep as shown in FIG. 16. The air-containing bag body 202 is attached to around the cervical vertebra, and the mandible holding unit 201 and the bag body 202 are coupled together via the magic tapes 213 and 223 (see FIG. 15).

When the cranial portion in a supine position is placed on the cranium supporting portion 220, the air in the cranium supporting portion 220 flows into the left and right protruding portions 221. In this case, the volume of the interior of the protruding portion 221 is expanded and the distal end of the protruding portion 221 is lifted upward and the mandible holding portions 210 coupled via the magic tapes 213 and 223 are also lifted upward. Since the mandible H1 is firmly held by the mandible holding portions 210, the mandible H1 is lifted relative to the cranial portion as the mandible holding portions 210 move.

As a result of the mandible H1 being lifted, the front side tissue (front wall T2 of the airway R1) of the airway R1 adhered to the submaxilla B1 is lifted with respect to the back side tissue of the airway R1 adhered to the vertebra B3 fixed to the cranial bone B2 and the airway R1 is expanded as shown in FIG. 5 and FIG. 6. For this reason, even when the lingual radix T1 sinks downward under the own weight thereof, the airway R1 is never closed and the airway R1 can be secured. As described above, it is possible to prevent a sleep apnea syndrome.

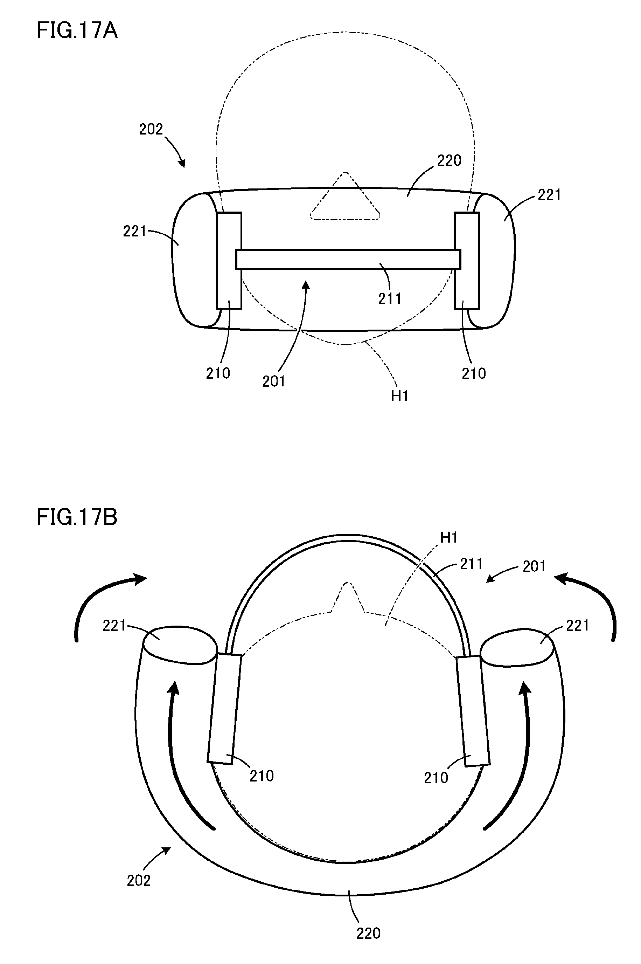

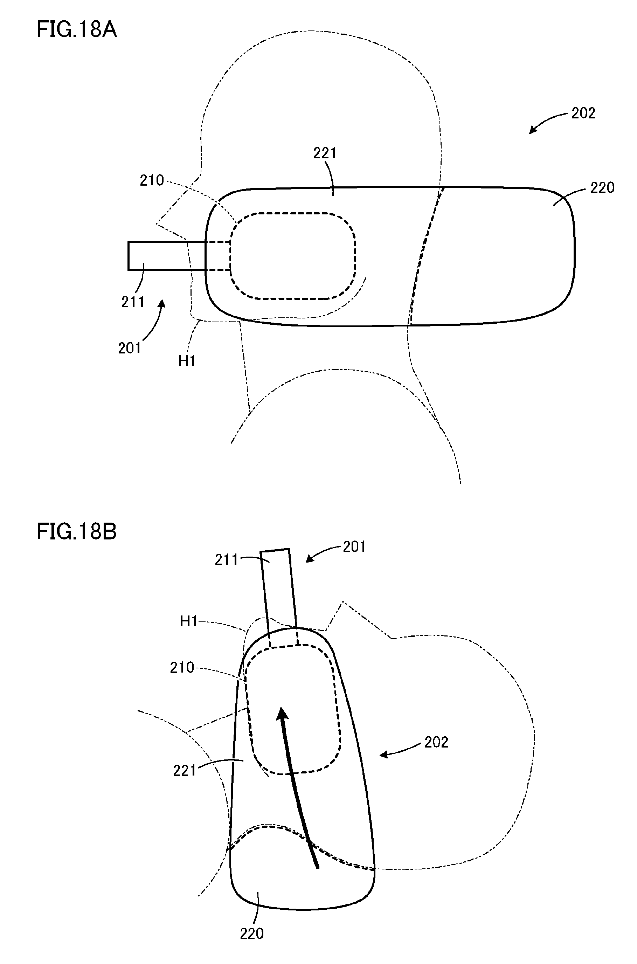

Next, an airway expansion operation will be described with reference to FIG. 17 and FIG. 18. FIG. 17 and FIG. 18 show a change of the shape of the bag body before and after lifting the mandible in the airway expansion apparatus according to the fourth modification example. FIG. 17 illustrates the airway expansion apparatus in FIG. 16 seen from the front or the lower limb direction and FIG. 18 illustrates the airway expansion apparatus in FIG. 16 seen from one side.

As shown in FIG. 17A and FIG. 18A, before placing the cranial portion on the cranium supporting portion 220, the interior of the bag body 202 is filled with air in such a way that the bag body 202 is not fully inflated, that is, the volume of the air filling the interior is smaller than the volume (capacity) of the bag body 202. When the user lies on his/her back and the cranial portion is placed on the cranium supporting portion 220, as shown in FIG. 17B and FIG. 18B, the cranium supporting portion 220 is crushed so as to follow the shape of the cranial portion or the cervical vertebra. When the cranium supporting portion 220 is crushed, the air in the cranium supporting portion 220 flows into the left and right protruding portions 221 (see the arrows in the drawing).

The bag body 202 is caused to bend inwardly (see the arrows in the drawing) around a predetermined location (to be more specific, substantially the center of the cranium supporting portion 220) of the cranium supporting portion 220 crushed by the cranial portion (see FIG. 17B). In this case, part of the air in the cranium supporting portion 220 flows from the cranium supporting portion 220 toward the protruding portions 221 at both ends and each protruding portion 221 is inflated, tensioned and lifted upward.

When the bag body 202 is bent inwardly and the mandible H1 is thereby sandwiched by the pair of protruding portions 221 from the left and right. Thus, the mandible H1 receives not only a holding force from the band portion 211 of the mandible holding unit 201 but also a holding force from the pair of protruding portions 221. In this way, the pair of protruding portions 221 also plays the role of assisting the holding force of the mandible holding portions 210. Furthermore, when the pair of protruding portions 221 are lifted upward, the mandible H1 held by the mandible holding portion 210 is likewise lifted upward (see FIG. 18B). As a result, as described above, the front side tissue of the airway adhered to the submaxilla is lifted with respect to the back side tissue of the airway and the airway is expanded.

Note that as described above, the volume of the region of the bag body 202 attached to the mandible holding portion 210 of the mandible holding unit 201 (the region corresponding to the protruding portion 221) is smaller than the volume of the region of the bag body 202 that supports the cranial portion (region corresponding to the cranium supporting portion 220). In this case, a sufficient amount of fluid moves from the region of the actuator supporting the cranial portion to the region attached to the mandible holding portion, and therefore the protruding portion is inflated and can lift the mandible holding portions upward. Thus, it is possible to implement effective airway expansion.

For example, the thickness of the protruding portion 221 in the left-right direction is smaller than the thickness of the cranium supporting portion 220 in the front-back direction. For this reason, the protruding portion 221 can be inflated and lift the mandible holding portions 210 even when the amount of air flow from the cranium supporting portion 220 is small. For example, even in the case of a user having a small-sized cranial portion, only placing the cranial portion on the cranium supporting portion 220 causes the pair of protruding portions 221 to inflate and can lift the mandible holding portion 210 upward.

Thus, by using the air as a fluid, it is possible to deform the bag body 202 in accordance with the size and shape of the user's cranial portion and the place of use. For this reason, it is possible to lift the mandible H1 regardless of the size and shape of the user's cranial portion and the place of use. Furthermore, as described above, the cranium supporting portion 220 keeps cushioning properties even when it is crushed by placing the cranial portion thereon and has a volume enough to stably support the cranial portion. For this reason, it is possible to keep the function as a pillow and prevent loss of sleeping comfort.

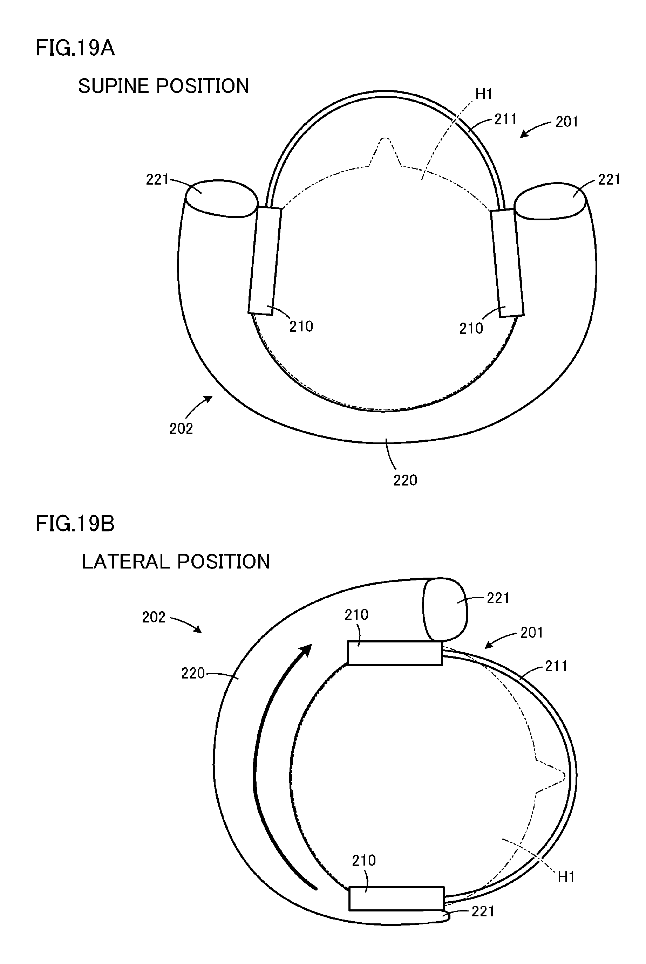

Next, operation of the airway expansion apparatus when the user changes the body position during sleep will be described with reference to FIG. 19. FIG. 19 is an operation diagram illustrating the airway expansion apparatus according to the fourth modification example when the user changes the body position. FIG. 19A shows the user in a supine position and FIG. 19B shows the user in a lateral position.

As shown in FIG. 19A, when the user is sleeping in a supine position, the pair of left and right protruding portions 221 are lifted upward and the user's airway is kept expanded. When the user turns over in his/her sleep and lies in a lateral position, one protruding portion 221 is located on the ground side (downward) as shown in FIG. 19B. In this case, the one protruding portion 221 is crushed by the weight of the cranial portion and the air in the protruding portion 221 flows into the cranium supporting portion 220 (see the arrow in the drawing).

As a result of the air in the protruding portion 221 flowing into the cranium supporting portion 220, the cranium supporting portion 220 slightly inflates on one hand and the protruding portion 221 contracts on the other. Therefore, the holding force of the mandible H1 by the pair of protruding portions 221 is weakened and the state in which the mandible H1 is lifted is canceled. Note that since the volume of the region of the bag body 202 attached to the mandible holding portion 210 of the mandible holding unit 201 (region corresponding to the protruding portion 221) is smaller than the volume of the region of the bag body 202 that supports the cranial portion (region corresponding to the cranium supporting portion 220), even when the air flows from the one protruding portion 220, the protruding portion 221 has no influence on a volume change of the cranium supporting portion 220 and the other protruding portion 220. Therefore, even when there is a postural change during sleep, this does not interfere the user's sleeping comfort.

When the user changes the body position to the supine position again, the air flows into the pair of protruding portions 221, the protruding portions 221 are lifted upward, and as a result, the mandible H1 is also lifted upward as shown in FIG. 19A. Thus, it is possible to change whether to lift the mandible H1 or not in accordance with the postural change of the user.

As described above, according to the airway expansion apparatus 200 according to the fourth embodiment, when the cranial portion is placed on the cranium supporting portion 220, the air (fluid) in the cranium supporting portion 220 flows into the pair of protruding portions 221, whereby the protruding portions 221 are inflated. This causes the protruding portions 221 to be lifted upward and the mandible holding portions 210 connected to the protruding portions 221 are also lifted upward together. Since the mandible H1 is held by the mandible holding portions 210, the mandible H1 is lifted upward as the protruding portions 221 inflate. In this case, the mandible H1 moves relative to the cranial portion and the airway is expanded. Thus, it is possible to use the air flow to cause the mandible holding unit 201 coupled to the bag body 202 to move and lift the mandible H1 held by the mandible holding unit 201, and thereby implement the airway expansion apparatus 200 in a low-cost configuration without requiring any complicated configuration.

In the above-described fourth modification example, the mandible holding unit 201 and the bag body 202 are configured to be detachable from each other, but the present invention is not limited to this configuration. The mandible holding unit 201 and the bag body 202 may be configured into a single unit.

The above-described fourth modification example adopts a configuration using air as a fluid, but the present invention is not limited to this configuration. The fluid may be any gas other than air or any material having fluid properties such as water, gel liquid or powder. Note that when water is used as the fluid, the bag body 202 can be used as a water pillow. In this case, the bag body 202 comes into contact around the user's neck and can cool around the neck.

In the above-described fourth modification example, the bag body 202 may be used alone as a pillow during sleep or a pillow may be provided separately and used with the bag body 202 placed thereon.

The above-described fourth modification example adopts a configuration in which the mandible holding unit 201 is attached to the mandible, but the present invention is not limited to this configuration. A configuration may be adopted in which an adhesive pad portion is attached to the pair of protruding portions 221, the mandible is held and lifted by only an air flow in the bag body 202. In this case, the pair of protruding portions 221 function as the mandible holding portion.

The above-described fourth modification example adopts a configuration in which the band portion 211 couples the pair of mandible holding portions 210, but the present invention is not limited to this configuration. The pair of mandible holding portions 210 need not be coupled by the band portion 211 if the mandible holding portions 210 can be kept in contact with the mandible.

The above-described fourth modification example adopts a configuration in which the mandible holding unit 201 and the bag body 202 are coupled via the magic tapes 213 and 223, but the present invention is not limited to this configuration. The configuration in which the mandible holding unit 201 and the bag body 202 are connected together may be any configuration if only the mandible holding unit 201 is detachable from the bag body 202, and the mandible holding unit 201 and the bag body 202 can be coupled together using, for example, a button or fastener.

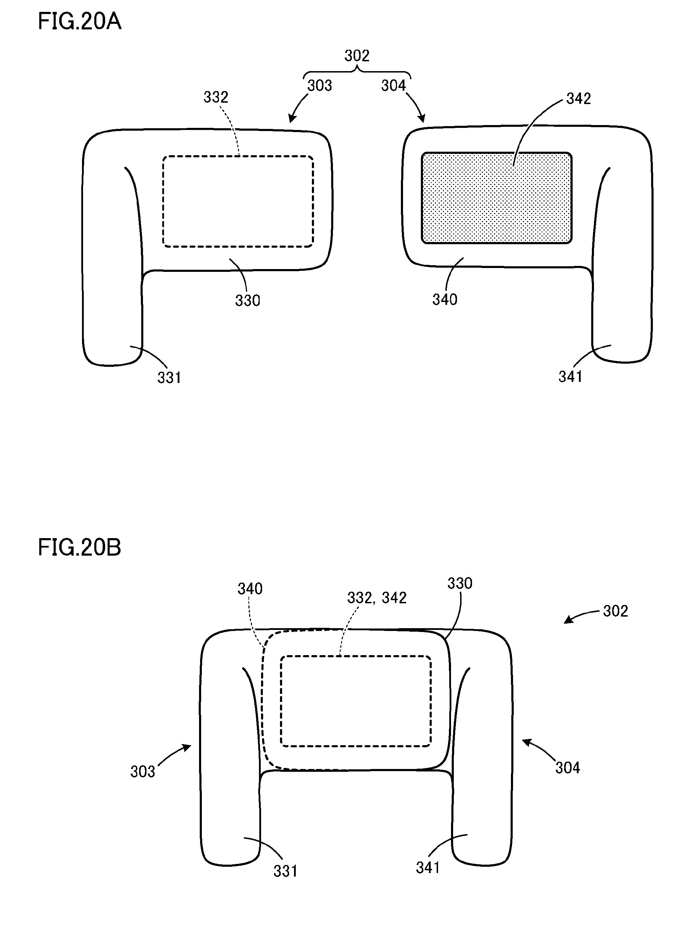

The above-described fourth modification example adopts a configuration in which the bag body 202 is formed into a U-figured single unit, but the present invention is not limited to this configuration. As shown in FIG. 20, the bag body 302 may have a shape separable as cranium supporting portions 330 and 340. In this case, the bag body 302 is configured by coupling a first bag body 303 and a second bag body 304 via magic tapes 332 and 342. The first bag body 303 and the second bag body 304 are horizontally symmetrical and roughly formed into an L-figured shape, and only different in positions at which the magic tapes 332 and 342 are attached. In the respective bag bodies 303 and 304, protruding portions 331 and 341 protrude toward the mandible from one end of the cranium supporting portions 330 and 340 that extend in leftward and rightward directions.

In this case, the cranium supporting portion 330 and the protruding portion 331, and the cranium supporting portion 340 and the protruding portion 341 are respectively configured to be able to contain air, the cranium supporting portion 330 and the protruding portion 331 internally communicate with each other and the cranium supporting portion 340 and the protruding portion 341 internally communicate with each other so that air can flow through the respective parts. Here, the volumes of regions of the first bag body 303 and the second bag body 304 attached to the mandible holding portions of the mandible holding unit (regions corresponding to the protruding portions 331 and 341) are preferably smaller than the volumes of regions of the first bag body 303 and the second bag body 304 that support the cranial portion (regions corresponding to the cranium supporting portions 330 and 340). Thus, when the cranial portion is placed on the first bag body 303 and the second bag body 304, the air in the bag body 202 is caused to flow under the own weight of the cranial portion, the bag body 202 is deformed and this deformation causes the mandible holding unit to move, and it is thereby possible to effectively implement an operation of lifting the mandible relative to the cranial portion.

The magic tape 332 is pasted to the underside of the cranium supporting portion 330 and the magic tape 342 is pasted to the surface side of the cranium supporting portion 340. Superimposing the cranium supporting portion 330 on the cranium supporting portion 340 causes the first bag body 303 to couple with the second bag body 304 into one bag body 302. For example, in the case of a configuration in which the bag body 302 and the mandible holding unit 201 (see FIG. 15) are integrated as one unit, it is possible to attach the mandible holding unit 201 to the mandible while the bag body 302 is decomposed, and then couple the cranium supporting portions 330 and 340 behind the cervical vertebra. Thus, compared to the configuration in which the bag body cannot be decomposed, it is possible to simplify the operation of attaching the airway expansion apparatus.

The above-described fourth modification example adopts a configuration in which the first bag body 303 and the second bag body 304 are coupled via the magic tapes 332 and 342, but the present invention is not limited to this configuration. The configuration in which the first bag body 303 and the second bag body 304 are connected together may be any configuration if only the first bag body 303 is detachable from the second bag body 304, and the first bag body 303 and the second bag body 304 can be coupled together using, for example, a button or fastener.

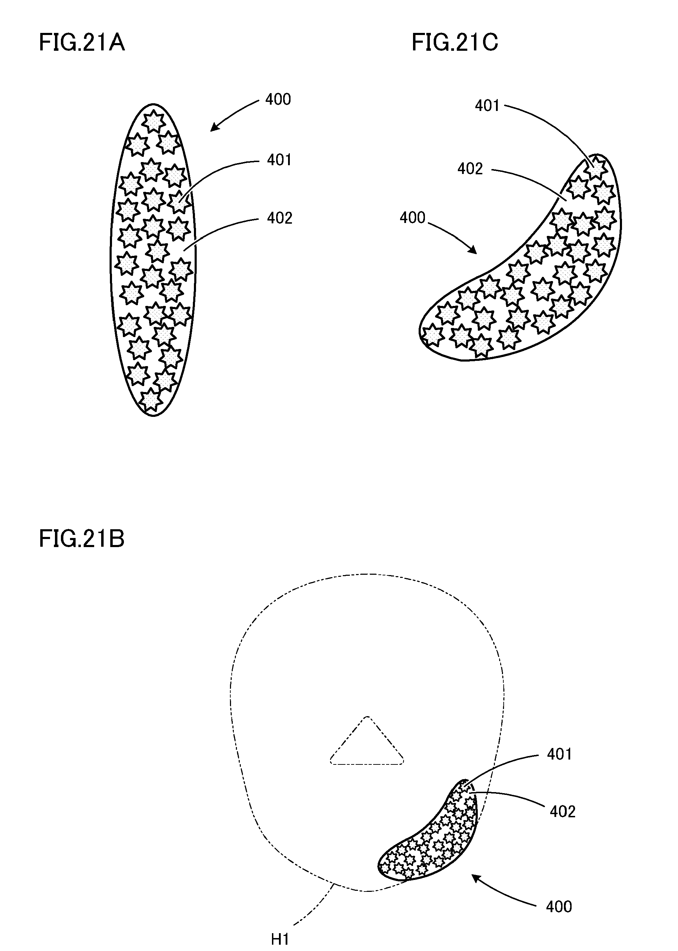

Furthermore, the pad (pad portion) is not limited to the above-described configuration, but the following configuration can also be adopted. Hereinafter, a modification example of the pad will be described with reference to FIG. 21. FIG. 21 is a diagram illustrating a modification example of the pad. The pad shown in FIG. 21 is a bag body filled with a mixture of plastic grains and an adhesive substance. First, FIG. 21A illustrates a completely unused pad. A bag body making up the pad 400 is filled with a mixture of plastic grains 401 and an adhesive substance 402 which is kept from hardening by being isolated from air or the like. FIG. 21B and FIG. 21C illustrate the unused pad 400 being held by the hand and pushed against the user's mandible. In this condition, the plastic grains 401 move inside the bag body and the pad 400 is deformed into a shape in close contact with the mandible H1. Afterwards, even when the pad 400 is detached from the mandible H1, the plastic grains 401 never move inside the bag body filled with the plastic grains 401 unless a force is added from outside and the pad 400 keeps the shape when it is pushed against the mandible H1. If the pad 400 is left in this condition, the adhesive substance 402 is hardened with passage of time, and the pad 400 is fixed in a shape in which it can be kept in close contact with the mandible H1. Since the mandible H1 can also be stably held by the mandible holding portions with the pad 400 kept in close contact with the mandible H1 in such a configuration, it is possible to effectively implement the operation of lifting the mandible H1.

### Industrial Applicability

The present invention has an effect of being able to prevent the lingual radix from falling into the airway and secure the airway without obstructing sleep and is applicable not only to patients in a hospital but also to drivers of public transportation during sleep who require sufficient sleep.

The present application is based on Japanese Patent Application No. 2013-163284 filed on August 6, 2013, entire content of which is expressly incorporated by reference herein.

## Claims

1. An airway expansion apparatus comprising:
a pillow portion that supports a cranial portion;
a pair of left and right mandible holding portions that hold a mandible coupled to the cranial portion at a temporomandibular joint;
a first actuation mechanism that causes the mandible holding portions to come into contact with the mandible; and
a second actuation mechanism that lifts the mandible holding portions with respect to the pillow portion with the mandible holding portions kept in contact with the mandible.

2. The airway expansion apparatus according to claim 1, further comprising pads in close contact with the mandible, wherein the mandible holding portions hold the mandible via the pads.

3. The airway expansion apparatus according to claim 2, wherein a contact surface of the pad in close contact with the mandible is curved so as to follow a corner of the mandible.

4. The airway expansion apparatus according to claim 2 or claim 3, wherein the contact surface of the pad in close contact with the mandible has adhesiveness.

5. The airway expansion apparatus according to any one of claims 1 to 4, wherein the mandible holding portions are fixed to the first actuation mechanism,
the first actuation mechanism is detachably connected to an actuator of the second actuation mechanism via the mandible holding portions, and
the first actuation mechanism can be removed from the second actuation mechanism by removing the mandible holding portions from the second actuation mechanism.

6. The airway expansion apparatus according to any one of claims 1 to 5, wherein the second actuation mechanism uses compressed air as a drive source.

7. The airway expansion apparatus according to any one of claims 1 to 5, further comprising:
a weight portion coupled to the mandible holding portions; and
a conversion mechanism that converts gravity of the weight portion into a direction in which the mandible holding portions are lifted,
wherein the gravity of the weight portion is used as a drive source.

8. The airway expansion apparatus according to any one of claims 1 to 7,
wherein the pillow portion comprises a cushioning portion that supports the cranial portion and a body portion that accommodates the cushioning portion and is formed into a U-figured shape so as to oscillate in a crosswise direction, and
the first actuation mechanism and the second actuation mechanism are attached to the body portion.

9. The airway expansion apparatus according to claim 8, wherein the second actuation mechanism comprises a switch mechanism that causes the pillow portion to support the cranial portion when the angle of oscillation of the body portion is within a predetermined angle range, switches, when the user is sleeping in a supine position, to an operating state in which the mandible holding portions are lifted in a lower limb and upward direction, where the direction in which the face is facing is assumed to be an upward direction, and switches, when the angle of oscillation of the body portion is outside the predetermined angle range, to a released state in which the operating state is released.

10. The airway expansion apparatus according to claim 9,
wherein the switch mechanism comprises a pair of left and right switches provided in correspondence with the predetermined angle range and a pendulum portion that oscillates between the pair of left and right switches, and
the released state is selected when the pendulum portion comes into contact with any one of the pair of left and right switches and the operating state is selected when the pendulum portion detaches from the pair of left and right switches.

11. An airway expansion apparatus comprising:
a pillow portion that supports a cranial portion;
a pair of left and right mandible holding portions that hold a mandible coupled to the cranial portion at a temporomandibular joint; and
a pair of left and right side plates in which slopes are formed so as to elevate from the mandible toward the parietal region in correspondence with the mandible holding portions,
wherein the mandible holding portion comprises an abutting portion abutting on the slope, and
the abutting portion moves the mandible holding portion in a direction perpendicular to the slope by abutting on the slope.

12. The airway expansion apparatus according to claim 3 or claim 4, wherein the pad is a bag body filled with a mixture of grains and an adhesive substance

13. An airway expansion apparatus comprising:
a mandible holding unit comprising a pair of mandible holding portions that hold a mandible coupled to a cranial portion at a temporomandibular joint and a coupling portion that couples the pair of mandible holding portions and keeps the mandible holding portions in contact with the mandible, and
an actuator that supports the cranial portion, is attached to the mandible holding portions and lifts the mandible holding portions with respect to the cranium supporting portion with the mandible holding portions kept in contact with the mandible,
wherein the actuator comprises a bag body whose interior can be filled with a fluid and the fluid filling the interior of the bag body can flow.

14. The airway expansion apparatus according to claim 12, wherein the actuator is attachable/detachable to/from the mandible holding unit.

15. The airway expansion apparatus according to claim 12 or claim 13, wherein the actuator is detachable.

16. The airway expansion apparatus according to any one of claims 12 to 14, wherein the volume of a region of the actuator attached to the mandible holding portions is smaller than the volume of a region of the actuator that supports the cranial portion.
